# EUROPEAN PATENT APPLICATION

(11) **EP 4 710 789 A1**
(43) Date of publication of application: **18.03.2026**
(21) Application number: 24806526.0
(22) Date of filing: 11.05.2024
(51) Int. Cl.: A24F 40/42, A24F 40/46, A24F 40/20

(54) **ATOMIZING MEDIUM ASSEMBLY AND AEROSOL GENERATING APPARATUS**

(30) Priority: 12.05.2023 CN 202310539624; 26.07.2023 CN 202310931987
(71) Applicant: Smoore International Holdings Limited, Grand Cayman, KY1-1111 (KY)
(72) Inventor: ZHENG, Wei, Shenzhen, Guangdong 518102 (CN); XIONG, Wei, Shenzhen, Guangdong 518102 (CN); CHEN, Daye, Shenzhen, Guangdong 518102 (CN); LUO, Yongjie, Shenzhen, Guangdong 518102 (CN)
(74) Representative: Winter, Brandl - Partnerschaft mbB
(86) International application number: PCT/CN2024/092726
(87) International publication number: WO 2024/235174

(57) **Abstract**

An atomization substrate assembly and an aerosol generating device are provided in embodiments of the present disclosure. The atomization substrate assembly includes a strip-shaped substrate and a housing assembly, where the housing assembly includes a housing, the housing is provided with a housing space for housing the strip-shaped substrate and includes a first housing and a second housing, one of the first housing and the second housing is provided with an opening for placing the strip-shaped substrate into the housing space, and the other one of the first housing and the second housing is arranged on the opening in a covering manner. According to the present disclosure, the demand of a user for a large number of times of atomization can be satisfied, frequent manual replacement with a new substrate is not required, and the user experience is better.

## Description

### TECHNICAL FIELD

The present disclosure relates to the technical field of atomization, and in particular to an atomization substrate assembly and an aerosol generating device.

### BACKGROUND

In the related art, a heat-not-burn device is primarily composed of an atomization assembly and an aerosol generating substrate. Due to the substrate difference, an aerosol including nicotine or cannabinol is generated to be inhaled by a user. However, the substrate can offer a small number of times of atomization each time, the user needs to manually change to a new substrate frequently, and thus the user experience is undesirable.

### SUMMARY

In view of the above, an atomization substrate assembly and an aerosol generating device are expected to be provided in embodiments of the present disclosure. Thus, a problem of the undesirable user experience caused by frequent manual replacement with a new substrate can be alleviated.

An atomization substrate assembly is provided in the embodiments of the present disclosure. The atomization substrate assembly includes:
a strip-shaped substrate; and
a housing assembly, where the housing assembly includes a housing, the housing is provided with a housing space for housing the strip-shaped substrate and includes a first housing and a second housing, one of the first housing and the second housing is provided with an opening for placing the strip-shaped substrate into the housing space, and the other one of the first housing and the second housing is arranged on the opening in a covering manner.

In some embodiments, the housing assembly is provided with an atomization cavity, the housing space includes a storage space and an accommodation space, and the storage space is spaced from the accommodation space;
the atomization substrate assembly further includes a feeding assembly, the feeding assembly includes a first reel arranged in the storage space and a second reel arranged in the accommodation space; and
the outer peripheral side of the first reel is used for winding the strip-shaped substrate, the strip-shaped substrate is unwound from the first reel and wound around the outer peripheral side of the second reel after passing through the atomization cavity, and the strip-shaped substrate is atomized to generate an aerosol in the atomization cavity.

In some embodiments, the atomization substrate assembly includes at least one guide member used for guiding the strip-shaped substrate to move.

In some embodiments, the housing assembly includes an atomization seat, the atomization seat is at least partially arranged in the housing and is provided with the atomization cavity and an air outlet channel communicating with the atomization cavity, and the air outlet channel is used for discharging the aerosol in the atomization cavity.

In some embodiments, the side wall of the side, in the thickness direction of the atomization substrate assembly, of the atomization seat is provided with a first avoiding port for the atomization assembly to extend, the portion, corresponding to the first avoiding port, of the housing is provided with a second avoiding port, and the second avoiding port is in butt joint with the first avoiding port.

In some embodiments, the housing is in a flat box shape, and the second housing and the first housing are in butt joint in the thickness direction and jointly define the storage space and the accommodation space; the feeding assembly is arranged at the bottom side of the atomization seat; the strip-shaped substrate moves in the atomization cavity in the transverse direction; and the transverse direction, the thickness direction, and the top-bottom direction are perpendicular to one another.

In some embodiments, the side, towards the first housing, of the atomization seat is opened and abuts against the inner surface of the first housing; the second housing is provided with a mounting port; and the side, farthest away from the first housing, of the atomization seat is arranged in the mounting port in a penetrating manner.

In some embodiments, the atomization seat includes a seat housing and a raised post protruding along the top surface of the seat housing in the direction towards the top side, the atomization cavity is defined by the seat housing, the raised post penetrates out of the top end surface of the housing, and the air outlet channel is at least partially defined by a space in the raised post.

In some embodiments, the housing assembly further includes a partition member, one end of the partition member is arranged at the inner surface of the first housing, and the other end extends in the thickness direction; the first housing and the second housing are in butt joint in the thickness direction and jointly define the storage space and the accommodation space along with the partition member; and the front end of the partition member at least forming the storage space is in sealing fit with the second housing.

In some embodiments, the atomization substrate assembly includes a first sealing member, where the first sealing member is sandwiched between the second housing and the front end of the partition member forming the storage space.

In some embodiments, the second housing is provided with a sealing recess, and the first sealing member is arranged in the sealing recess.

In some embodiments, the side wall of the storage space is provided with an outlet, and the portion, in the storage space, of the strip-shaped substrate moves out of the storage space through the outlet; and
the atomization substrate assembly includes a second sealing member arranged at the outlet, the second sealing member is used for sealing the outlet, and the strip-shaped substrate and the second sealing member are in sealing fit and are movable relative to each other.

In some embodiments, the second sealing member is provided with a through recess, and the strip-shaped substrate is movably arranged the through recess in a penetrating manner and is in sealing fit with the recess wall of the through recess.

In some embodiments, the first housing is detachably or rotatably connected to the second housing.

In some embodiments, the atomization substrate assembly further includes a guide assembly used for guiding the strip-shaped substrate from the first reel to the atomization cavity and the second reel at one time; and the guide assembly includes at least two guide members, and the portion, between the two guide members, of the strip-shaped substrate is arranged in the atomization cavity in a penetrating manner.

In some embodiments, the guide members and the feeding assembly are arranged on the first housing separately, and the positions, corresponding to the guide members, of the second housing are respectively provided with limiting structures used for preventing the strip-shaped substrate from being separated.

In some embodiments, the first housing or the second housing is further provided with at least one connection structure, and the connection structure includes connection holes provided in correspondence to the first reel and/or the second reel.

In some embodiments, the first housing and/or the second housing are/is further provided with pouring ports/a pouring port, and the pouring port communicates with the accommodation space.

In some embodiments, the housing assembly further includes an ash collection member, and the ash collection member is detachably or rotatably arranged on the pouring port in a covering manner.

In some embodiments, the atomization substrate assembly includes an atomization assembly arranged at the housing assembly, and the atomization assembly at least partially extends into the atomization cavity to be used for atomizing the strip-shaped substrate.

In some embodiments, the atomization assembly includes a heating member, and the heating member extends into the atomization cavity to be in contact with the strip-shaped substrate or spaced from the strip-shaped substrate in parallel.

In some embodiments, the portion, extending into the atomization cavity, of the heating member corresponds to the portion, in the atomization cavity, of the strip-shaped substrate in the width direction and/or is centrally arranged in the atomization cavity in the length direction.

An aerosol generating device is configured by the present disclosure. The device includes:
a main machine, where the main machine includes a machine case, a driving assembly, and a power supply assembly, and the power supply assembly is arranged in the machine case; and
any atomization substrate assembly described above, where the atomization substrate assembly is detachably arranged in the machine case, and the driving assembly partially extends into the atomization substrate assembly to be at least in drive connection to a second reel.

In some embodiments, a housing assembly includes an atomization seat, the atomization seat is at least partially arranged in a housing and is provided with an atomization cavity and an air outlet channel communicating with the atomization cavity, and the air outlet channel is used for discharging an aerosol in the atomization cavity;
the side wall of the side, in the thickness direction of the atomization substrate assembly, of the atomization seat is provided with a first avoiding port for an atomization assembly to extend, the portion, corresponding to the first avoiding port, of the housing is provided with a second avoiding port, and the second avoiding port is in butt joint with the first avoiding port; and
the main machine further includes the atomization assembly, the atomization assembly includes a heating member and a connection plate connected to the heating member, the heating member extends into the atomization cavity to be in at least partial contact with a strip-shaped substrate, and the connection plate closes the second avoiding port or the first avoiding port.

In some embodiments, the connection plate is detachably connected with the housing or the side wall of the atomization cavity; and/or
the atomization assembly includes a connector electrically connected to the heating member, the connector is arranged at the side, facing away from the atomization cavity, of the connection plate, and the connector is used for being electrically connected to the power supply assembly.

The atomization substrate assembly according to the embodiments of the present disclosure includes the first housing and the second housing. The first housing and the second housing jointly define the housing space to accommodate the strip-shaped substrate. Moreover, one of the first housing and the second housing is provided with the opening for placing the strip-shaped substrate into the housing space, and the other one of the first housing and the second housing is arranged on the opening in the covering manner. Thus, mutual communication between the housing space and the outside can be operably implemented. When to be replaced, the strip-shaped substrate can be moved out only by removing the second housing from the opening. Moreover, a large quantity of the strip-shaped aerosol generating substrate can be accommodated in the housing space. Thus, the demand of a user for a large number of times of atomization is satisfied, and frequent manual replacement with a new substrate is not required. Moreover, the strip-shaped substrate has the uniform thickness, so that the consistency of atomization can be ensured, and the user experience is better.

Also, the first housing and the second housing are arranged to further solve a storage problem of the aerosol generating substrate, and no storage structure needs to be additionally arranged to store the aerosol generating substrate. The relatively closed housing space can accommodate and protect the strip-shaped substrate, and external humid air is prevented from affecting the aerosol generating substrate, so that the service life and the storage duration of the aerosol generating substrate are prolonged.

### BRIEF DESCRIPTION OF THE DRAWINGS

FIG. 1 is a schematic structural diagram of an aerosol generating device according to a first embodiment of the present disclosure;
FIG. 2 is an exploded view of the aerosol generating device shown in FIG. 1;
FIG. 3 is a schematic structural diagram of a section of the aerosol generating device shown in FIG. 1;
FIG. 4 is a schematic structural diagram of the aerosol generating device shown in FIG. 1 without an outer cover;
FIG. 5 is a schematic structural diagram of an atomization assembly of the aerosol generating device in FIG. 1;
FIG. 6 is a schematic structural diagram of an atomization substrate assembly in FIG. 2;
FIG. 7 is an exploded view of the atomization substrate assembly shown in FIG. 6;
FIG. 8 is a schematic structural diagram of an atomization seat in FIG. 6;
FIG. 9 is an exploded view of an aerosol generating device according to a second embodiment of the present disclosure;
FIG. 10 is a schematic structural diagram of an atomization substrate assembly in FIG. 9;
FIG. 11 is an exploded view of the atomization substrate assembly in FIG. 10;
FIG. 12 is a schematic structural diagram of a section of an aerosol generating device according to a third embodiment of the present disclosure;
FIG. 13 is a schematic structural diagram of a section of an aerosol generating device according to a fourth embodiment of the present disclosure;
FIG. 14 is a schematic structural diagram of an atomization substrate assembly according to a fifth embodiment of the present disclosure;
FIG. 15 is an exploded view of the atomization substrate assembly shown in FIG. 14;
FIG. 16 is a schematic structural diagram of a part of an atomization substrate assembly according to a sixth embodiment of the present disclosure;
FIG. 17 is a schematic structural diagram of a part of an atomization substrate assembly according to a seventh embodiment of the present disclosure;
FIG. 18 is a schematic structural diagram of a part of an atomization substrate assembly according to an eighth embodiment of the present disclosure;
FIG. 19 is a schematic structural diagram of a second housing of the atomization substrate assembly shown in FIG. 18;
FIG. 20 is a schematic structural diagram of a second sealing member of the atomization substrate assembly shown in FIG. 18;
FIG. 21 is a schematic structural diagram of a part of an atomization substrate assembly according to a ninth embodiment of the present disclosure;
FIG. 22 is a schematic structural diagram of a part of an atomization substrate assembly according to a tenth embodiment of the present disclosure;
FIG. 23 is a schematic structural diagram of a part of an atomization substrate assembly according to an eleventh embodiment of the present disclosure; and
FIG. 24 is a schematic structural diagram of a part of an atomization substrate assembly according to a twelfth embodiment of the present disclosure.

### DETAILED DESCRIPTION

Implementations of the present disclosure are further described in detail below with reference to the accompanying drawings and embodiments. The following embodiments are used to illustrate the present disclosure, and cannot limit the scope of the present disclosure.

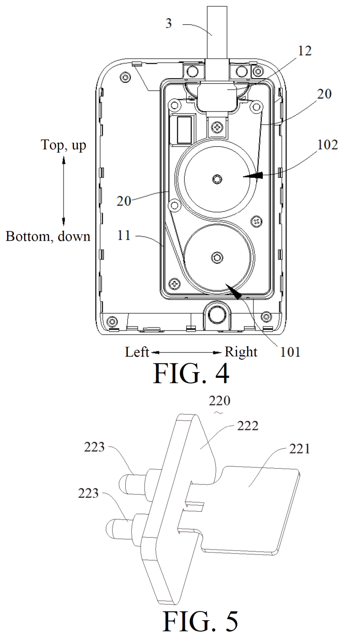

In the descriptions in the embodiments of the present disclosure, it should be noted that the orientation or position relationships indicated by the terms such as "up", "down", "front", "rear", "left", "right", "top", "bottom", etc. are based on the orientation or position relationships shown in FIG. 4, FIG. 7, and FIG. 24. The term "height direction" indicates the up-down direction, the term "thickness direction" indicates the front-rear direction, and the term "transverse direction" indicates the left-right direction. The terms are merely used for ease and brevity of the description of the embodiments of the present disclosure, rather than indicating or implying that the mentioned device or element must have a particular orientation or be constructed and operated in a particular orientation, and thus cannot be interpreted as limiting the embodiments of the present disclosure. In addition, the terms "first", "second" and "third" are for descriptive purposes only and should not be construed as indicating or implying relative importance.

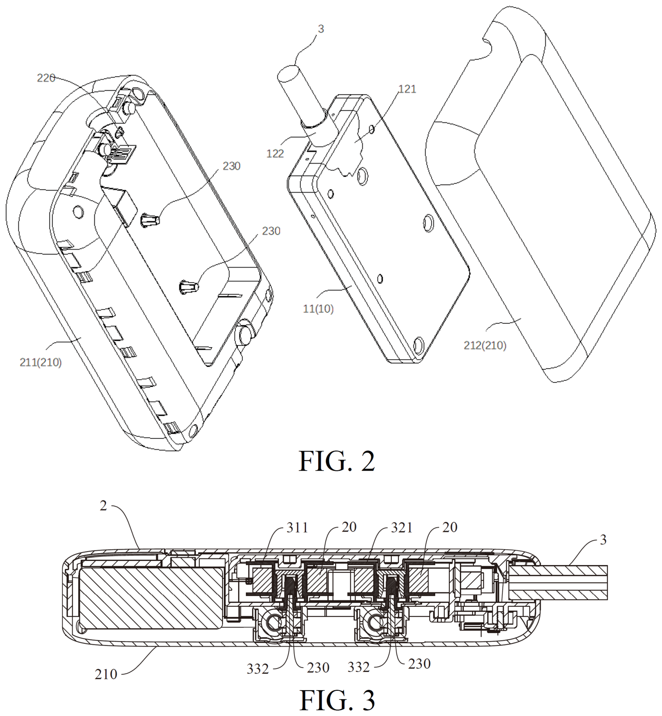

An atomization substrate assembly for a strip-shaped substrate is provided in the embodiments of the present disclosure. As shown in FIG. 2 to FIG. 8, the atomization substrate assembly 1 may be used for accommodating the strip-shaped substrate 20.

The atomization substrate assembly 1 includes the strip-shaped substrate 20.

The atomization substrate assembly 1 includes a housing assembly 10 and a feeding assembly 30.

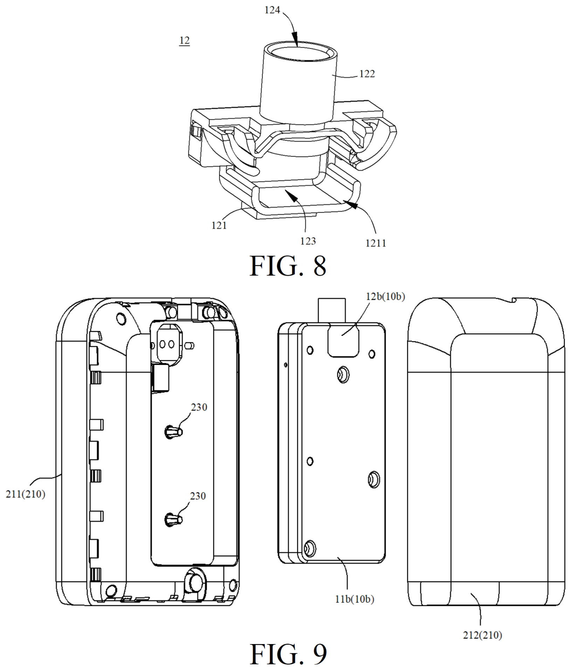

With reference to FIG. 8, the housing assembly 10 includes an atomization cavity 123. The atomization cavity 123 communicates with the outside. The strip-shaped substrate 20 may be atomized to generate an aerosol in the atomization cavity 123. The aerosol generated in the atomization cavity 123 may be discharged to be inhaled by a user.

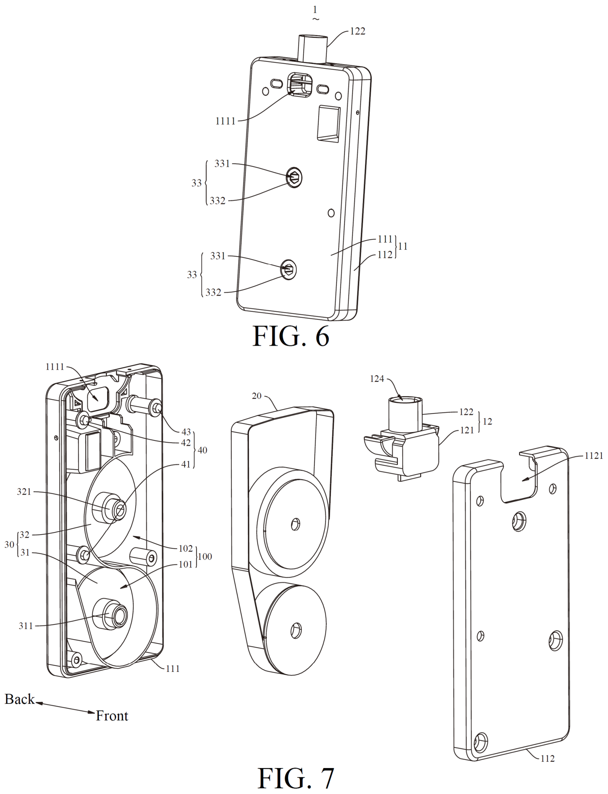

With reference to FIG. 4 and FIG. 7, the feeding assembly 30 is used for conveying the strip-shaped substrate 20 that can generate the aerosol, so that the strip-shaped substrate 20 moves in the atomization substrate assembly 1 to generate the aerosol to be inhaled by the user in the atomization cavity 123.

The housing assembly 10 is provided with a housing space 100 used for housing the strip-shaped substrate 20. The housing space 100 includes a storage space 101 and an accommodation space 102. The feeding assembly 30 includes a first reel 311 arranged in the storage space 101 and a second reel 321 arranged in the accommodation space 102. The outer peripheral side of the first reel 311 is used for winding the strip-shaped substrate 20. The strip-shaped substrate 20 may be unwound from the first reel 311 and wound around the outer peripheral side of the second reel 321 after passing through the atomization cavity 123. To be specific, a non-atomized strip-shaped substrate 20 is unwound from the first reel 311 and wound around the second reel 321 after atomization.

Illustratively, two ends of the strip-shaped substrate 20 are wound around the outer periphery of the first reel 311 and the outer periphery of the second reel 321 respectively. The second reel 321 is controlled to rotate, so as to drive the portion, wound around the first reel 311, of the strip-shaped substrate 20 to pass through the atomization cavity 123 and to be wound around the second reel 321. The rotation direction of the second reel 321 may be the clockwise direction or the anticlockwise direction.

Unwinding is to remove a coiled substrate in the radial direction, so that the outer layer of the substrate is separated from the inner layer of the substrate. The strip-shaped substrate 20 is wound around the outer peripheral side of the first reel 311. The strip-shaped substrate 20 may be unwound from the first reel 311. This indicates a process of separating the outer layer of the strip-shaped substrate 20 from the inner layer of the strip-shaped substrate 20.

Winding is to wind a continuous article through a spool, a reel, etc. After passing through the atomization cavity 123, the strip-shaped substrate 20 is wound around the outer peripheral side of the second reel 321 and is wound by the second reel 321. This indicates a process of winding the atomized strip-shaped substrate 20 into a coil through the second reel 321.

Specifically, the strip-shaped substrate 20 is of a flexible elongated structure that has the particular width and thickness, and can be extended or bent in the length direction according to an actual situation.

In the embodiment, the section of the strip-shaped substrate 20 is approximately in a flat rectangle shape.

In some other optional embodiments, the section of the strip-shaped substrate 20 is of the elongated structure in a circular shape, a polygonal shape, an irregular shape, etc.

The aerosol generating substrate used for generating the aerosol coats the surface of the strip-shaped substrate 20 or is infiltrated or embedded into the strip-shaped substrate. The aerosol generating substrate includes, but is not limited to, a medicine, a material including nicotine, etc.

Illustratively, the strip-shaped substrate 20 may include a base band and an aerosol generating substrate alternately spread on the base band in a spaced manner.

The base band may be any strip capable of withstanding the atomization temperature, for example, a paper strip and a polymer strip. It has flexibility and can be unwound and wound, for example, a metal base band or a graphite base band. The base band may alternatively be a metal mesh. The metal mesh may be formed by weaving a metal wire, or may be formed by providing a plurality of through holes in a metal sheet.

The strip-shaped substrate 20 is arranged in the housing assembly 10 and connected to the feeding assembly 30. The feeding assembly 30 is also arranged in the housing assembly 10 and used for driving the strip-shaped substrate 20 to move in the atomization cavity 123.

The strip-shaped substrate 20 is wound into the coil and mounted at the outer peripheral side of the first reel 311 as shown in FIG. 7. The end portion of the strip-shaped substrate 20 is pulled out of the coil and fixed to the outer peripheral side of the second reel 321 after passing through the atomization cavity 123. The above operations can be completed before delivery of the atomization substrate assembly 1 for replacement and mounting by the user. The strip-shaped substrate may alternatively be sold separately to be assembled by the user.

Illustratively, in some embodiments, with reference to FIG. 2, FIG. 4, and FIG. 7, the housing assembly 10 includes a housing 11 and an atomization seat 12. The atomization seat 12 is at least partially arranged in the housing 11. The housing 11 is provided with the storage space 101 and the accommodation space 102. The atomization seat 12 is provided with the atomization cavity 123 and an air outlet channel 124 communicating with the atomization cavity 123. The air outlet channel 124 is used for discharging the aerosol in the atomization cavity 123. To be specific, the strip-shaped substrate 20 is heated and atomized to generate the aerosol in the atomization cavity 123, and the aerosol in the atomization cavity 123 is discharged through the air outlet channel 124, to be inhaled by the user. Understandably, the air outlet channel 124 may be integrally formed with the atomization cavity 123 or may be a separate member connected to the atomization cavity 123. The air outlet channel 124 may be detachably or fixedly connected to the atomization cavity 123.

In some other embodiments, the housing assembly 10 may alternatively not include the atomization seat 12. To be specific, the atomization cavity 123 is formed by the housing assembly 10 instead of the atomization seat 12.

Understandably, the atomization seat 12 is at least partially arranged in the housing 11. To be specific, some structures of the atomization seat 12 are arranged in the housing 11, or all structures of the atomization seat 12 are arranged in the housing 11. The housing 11 provides a mounting space for the atomization seat 12 and supports and protects the atomization seat 12.

The atomization seat 12 is independently provided with the atomization cavity 123 and the air outlet channel 124. The air outlet channel 124 communicates with the atomization cavity 123 and is used for discharging the aerosol in the atomization cavity 123.

It should be noted that the atomization seat 12 herein is independently provided with the atomization cavity 123 and the air outlet channel 124. This indicates that the atomization seat 12 is provided with the independent atomization cavity 123 and the independent air outlet channel 124 based on the structure or shape design of the atomization seat 12.

As shown in FIG. 6 and FIG. 7, the housing 11 is in a flat box shape, and includes a first housing 111 and a second housing 112. The first housing 111 and the second housing 112 jointly define the housing space 100. Moreover, one of the first housing 111 and the second housing 112 is provided with an opening for placing the strip-shaped substrate 20 into the housing space 100, and the other one of the first housing and the second housing is arranged on the opening in a covering manner, to isolate the housing space 100 from the outside.

In the embodiment, the second housing 112 and the first housing 111 are in butt joint in the thickness direction, and jointly define the housing space 100. The first housing and the second housing are in a flat rectangle shape and close the housing space 100 through fastening, etc. Thus, it may be deemed that the second housing 112 is provided with the opening or the first housing 111 is provided with the opening (not marked in the figure).

Through the structural design of the housing 11, a large mounting space can be provided for the atomization seat 12 and the feeding assembly 30. Through the positional arrangement of the feeding assembly 30, the atomization seat 12, and the strip-shaped substrate 20, and the flat arrangement of the aerosol generating device, the small thickness can be maintained, proper layout of various members can be achieved, and thus the structural layout inside the atomization substrate assembly 1 can be more proper.

In some embodiments, to facilitate assembly and replacement of the strip-shaped substrate 20, the first housing 111 is detachably connected to the second housing 112. To be specific, when the strip-shaped substrate 20 is to be assembled or replaced, in a case that the first housing 111 is separated from the second housing 112, after the strip-shaped substrate 20 is assembled or replaced between the first housing 111 and the second housing 112, the other one of the first housing 111 and the second housing 112 is assembled. Illustratively, the first housing 111 may be connected to the second housing 112 through magnetic attraction, snap fit, threads, insertion, ultrasound, etc.

In some embodiments, a sealing member is further arranged between the first housing 111 and the second housing 112, to seal a gap between the first housing 111 and the second housing 112. Thus, relative sealing performance inside the housing space 100 is ensured, the strip-shaped substrate 20 inside is prevented from being affected by a humid environment, etc., damage, off-odor, etc. of the strip-shaped substrate 20 are avoided, and the service life of the strip-shaped substrate 20 is prolonged.

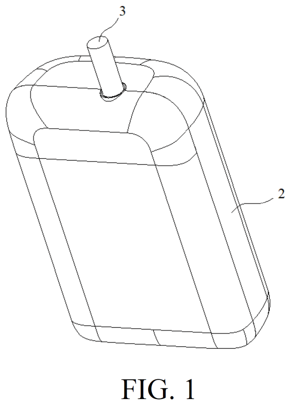

An aerosol generating device is provided in the embodiments of the present disclosure. With reference to FIG. 1 to FIG. 4, the device includes a main machine 2 and the atomization substrate assembly 1 according to any embodiment of the present disclosure.

As shown in FIG. 2 and FIG. 3, the main machine 2 includes a machine case 210, where the atomization substrate assembly 1 is detachably arranged in the machine case 210. To be specific, a new strip-shaped substrate 20 is assembled into the atomization substrate assembly 1 to form the atomization substrate assembly 1, and then the atomization substrate assembly 1 with the new strip-shaped substrate 20 is assembled to the machine case 210, to be used by the user. After the strip-shaped substrate 20 in the atomization substrate assembly 1 is used up, the atomization substrate assembly 1 may be disassembled from the main machine 2 and replaced with a new atomization substrate assembly 1. Alternatively, the atomization substrate assembly 1 may be first disassembled from the main machine 2, and then the used strip-shaped substrate is replaced with a new strip-shaped substrate 20, so that the use cost of the user is reduced.

Illustratively, with reference to FIG. 2, the main machine 2 includes a body 211 and an outer cover 212 arranged on the body 211 in a covering manner. The body 211 includes a mounting space with a mounting port, and the outer cover 212 is arranged on the mounting port in a covering manner.

To facilitate maintenance or replacement of the atomization substrate assembly 1, the atomization substrate assembly 1 needs to be assembled and disassembled conveniently, and the body 211 is detachably or rotatably connected to the outer cover 212. Illustratively, the body 211 may be connected to the outer cover 212 through magnetic attraction, snap fit, threads, insertion, ultrasound, etc.

The main machine 2 includes a power supply assembly and an atomization assembly 220. The power supply assembly is arranged in the machine case 210. The atomization assembly 220 is electrically connected to the power supply assembly and used for atomizing the strip-shaped substrate 20. Illustratively, at least some structures of the atomization assembly 220 extend into the atomization cavity 123 and are used for atomizing the portion, located in the atomization cavity 123, of the strip-shaped substrate 20. For example, a heating member 221 of the atomization assembly 220 extends into the atomization cavity 123 and is used for atomizing the portion, located in the atomization cavity 123, of the strip-shaped substrate 20.

The power supply assembly is non-detachably connected to the machine case 210, and the power supply assembly may not be discarded until used up. Alternatively, the power supply assembly is detachably connected to the machine case 210. To be specific, the power supply assembly may be detachable, replaceable, or re-chargeable (inside or outside the aerosol generating device).

As shown in FIG. 2 and FIG. 3, in some embodiments, the main machine 2 further includes at least one driving assembly 230. The driving assembly 230 at least partially extends into the atomization substrate assembly 1 to be at least in drive connection to the second reel 321. In other words, the driving assembly 230 may drive the second reel 321. The driving assembly 230 is arranged on the main machine 2. When the atomization substrate assembly 1 is assembled to the machine case 210, the driving assembly 230 partially extends into the atomization substrate assembly 1 to be in drive connection to the second reel 321.

Illustratively, in some embodiments, the driving assembly 230 may be in drive connection to only the second reel 321. In some other embodiments, the driving assembly 230 may alternatively be in drive connection to the first reel 311 and the second reel 321.

The driving assembly 230 drives the strip-shaped substrate 20 wound around the first reel 311 to pass through the atomization cavity 123 to be wound around the second reel 321 by driving the second reel 321 to rotate.

As shown in FIG. 3, in the embodiment, two driving assemblies 230 are provided and used for driving the first reel 311 and the second reel 321 to rotate respectively. The two driving assemblies 230 are used for simultaneously driving the first reel 311 and the second reel 321 to synchronously rotate. Thus, the strip-shaped substrate 20 is prevented from being stretched during rotation, the stress on the strip-shaped substrate 20 is reduced, and the strip-shaped substrate is protected.

When the user needs to inhales the aerosol, the second reel 321 rotates under driving by the driving assembly 230, and the first reel 311 rotates under driving by the other driving assembly 230. The first reel 311 continuously unwinds the strip-shaped substrate 20 from the first reel 311. The unwound strip-shaped substrate 20 is atomized by the atomization assembly 220 in the atomization cavity 123 along the way. The aerosol generated in the atomization cavity 123 is discharged to be inhaled by the user. The atomized strip-shaped substrate 20 is wound around the second reel 321. When the content of the aerosol generating substrate on the surface of or inside the portion, in the atomization cavity 123, of the strip-shaped substrate 20 is gradually reduced, the driving assembly 230 drives the feeding assembly 30 to drive the strip-shaped substrate 20 to move, so that the used portion, located in the atomization cavity 123, of the strip-shaped substrate 20 is moved out of the atomization cavity 123, and the unused portion of the strip-shaped substrate 20 is moved into the atomization cavity 123 to be heated and atomized by the heating member 221 of the atomization assembly 220. Thus, the aerosol can continue to be generated to be inhaled by the user. In respond to an operation by the user in real time, the aerosol generating device may be activated at any time, continuously supply and atomize a large number of the strip-shaped substrate 20, and stop supply and atomization at any time, almost without response time delay. After the user inhales the aerosol for particular time or a particular number of times, the strip-shaped substrate 20 coil is used up. The user may open the main machine 2 to take out the atomization substrate assembly 1 for replacement, or may further open the atomization substrate assembly 1 for replacement with a next strip-shaped substrate 20 coil.

In the related art, a heat-not-burn device is primarily composed of the atomization assembly and the aerosol generating substrate. The aerosol generating substrate is, for example, a liquid aerosol generating substrate or a conventional replaceable cigarette. A single cigarette can offer a small number of times of atomization, and thus the user needs to perform frequent replacement with a processed replaceable cigarette.

The atomization substrate assembly 1 according to the embodiments of the present disclosure includes the housing assembly 10 and the feeding assembly 30. The housing assembly 10 is provided with the atomization cavity 123, the storage space 101, and the accommodation space 102. The feeding assembly 30 includes the first reel 311 arranged in the storage space 101 and the second reel 321 arranged in the accommodation space 102. The two ends of the strip-shaped substrate 20 used for generating the aerosol are wound around the first reel 311 and the second reel 321 respectively. When an effective component in the strip-shaped substrate 20 is gradually reduced during use, the second reel 321 and the first reel 311 are rotated, so that the unused portion, around the first reel 311, of the strip-shaped substrate 20 is conveyed into the atomization cavity 123 to be atomized to generate the aerosol. To be specific, the atomized portion, located in the atomization cavity 123, of the strip-shaped substrate 20 may be moved out of the atomization cavity 123, and the non-atomized portion of the strip-shaped substrate 20 is moved into the atomization cavity 123 to be atomized to generate the aerosol, so as to be used by the user. In other words, the strip-shaped substrate 20 is wound around the outer peripheral side of the first reel 311 in the storage space 101, and the strip-shaped substrate 20 wound into the coil can satisfy the demand of the user for a large number of times of atomization. Moreover, the strip-shaped substrate 20 has the uniform thickness, and thus the consistency of atomization can be ensured, and the user experience is better. In addition, the unused strip-shaped substrate 20 is wound around the first reel 311, and the first reel 311 can protect the strip-shaped substrate 20. For example, the strip-shaped substrate 20 wound into the coil can reduce the probability of air drying or oxidation and prolong the service life.

As further shown in FIG. 3 and FIG. 6, in some embodiments, the feeding assembly 30 is provided with at least one connection structure 33. The connection structure 33 is arranged on the first housing 111 and used for causing the driving assembly 230 to drive the second reel 321 and/or the first reel 311 to rotate.

Specifically, the connection structure 33 indicates connection holes 331 formed in the first housing 111. Two connection holes 331 are provided in correspondence to the first reel 311 and the second reel 321 respectively. Output shafts of the two driving assemblies 230 are arranged in the two connection holes 331 respectively in a penetrating manner and connected to the second reel 321 through the connection holes 331, to drive the second reel 321 to rotate.

In the embodiment, in addition to the connection holes 331, the connection structure 33 further includes connection members 332 arranged in the connection holes 331. The connection members 332 are rotatably arranged in the connection holes 331. The driving assembly 230 and the second reel 321 are connected to the connection members 332 separately. The driving assembly 230 drives the second reel 321 to rotate through the connection member 332.

Specifically, the connection member 332 is arranged in a barrel shape with at least one penetrated-through end and arranged in the first reel 32 or the second reel 31 in a penetrating manner. The driving assembly 230 partially penetrates the connection hole 331 to extend into the connection member 332, so as to be connected to the connection member 332. Further, the connected portion of the driving assembly 230, the connection member 332, and the first reel 311 (or the second reel 321) are coaxially arranged, so that the driving assembly 230 drives the connection member 332 and the first reel 311 (or the second reel 321) to rotate.

In some other optional embodiments, the connection structure 33 may alternatively be arranged on the second housing 112. In some embodiments, with reference to FIG. 4, the feeding assembly 30 is arranged at the bottom side of the atomization seat 12, and the strip-shaped substrate 20 moves in the atomization cavity 123 in the transverse direction (the left-right direction). For example, the atomization seat 12 is arranged at the top of the housing assembly 10, and the feeding assembly 30 is arranged at the bottom side of the atomization seat 12, where the transverse direction, the thickness direction, and the top-bottom direction are perpendicular to one another. For example, the atomization cavity 123, the accommodation space 102, and the storage space 101 are linearly arranged in the top-bottom direction. Thus, the space in the housing assembly 10 can be fully used, the size of the atomization substrate assembly 1 is reduced to a particular extent, and the user experience is better.

The feeding assembly 30 is arranged at the bottom side of the atomization seat 12, so that the atomization cavity 123 is located at one end of the housing space 100 (the housing space 100 is located at the bottom side of the atomization cavity 123). Specifically, the first reel 311 and the second reel 321 are arranged at the bottom side of the atomization seat 12. After the aerosol generating substrate on the surface of or inside the strip-shaped substrate 20 is heated and atomized to generate the aerosol in the atomization cavity 123, the feeding assembly 30 drives the strip-shaped substrate 20 to move. The used portion, in the atomization cavity 123, of the strip-shaped substrate 20 may move towards the bottom side to the second reel 321. The unused portion, around the first reel 311, of the strip-shaped substrate 20 may move into the atomization cavity 123 from the bottom side. Thus, the movement of the strip-shaped substrate 20 is basically located at the bottom side of the atomization seat 12 and does not affect the atomization seat 12. The atomization seat 12 is arranged in the direction closer to the top side. In this way, the aerosol in the atomization cavity 123 can also be rapidly conveyed to the outside to be inhaled by the user.

In the embodiment, the atomization substrate assembly 1 is vertically arranged in the main machine 2, and the axis of the housing 11 in the length direction is parallel to the axis of the machine case 210 of the main machine 2 in the top-bottom direction. In this way, the atomization seat 12 is arranged on the side wall of the housing 11 in the width direction (i.e. the left-right direction in FIG. 4). The second reel 321 is located between the first reel 311 and the atomization seat 12, and the connection line among the first reel, the second reel, and the atomization seat is approximately linear and approximately coincides with the axis of the housing 11.

In some other optional embodiments, the first reel 311 may be located between the second reel 321 and the atomization seat 12, and the atomization seat 12 may alternatively be located between the first reel 311 and the second reel 321. The connection line among the first reel, the second reel, and the atomization seat may not be linear and may alternatively not be parallel to or coincide with the axis of the housing 11.

Illustratively, with reference to FIG. 7 and FIG. 8, the side, towards the first housing 111, of the atomization seat 12 is opened and abuts against the inner surface of the first housing 111. The second housing 112 is provided with a mounting port 1121. The side, farthest away from the first housing 111, of the atomization seat 12 is arranged in the mounting port 1121 in a penetrating manner.

The first housing 111 may support the atomization seat 12.

Specifically, in the embodiment, the mounting port 1121 is in a recess shape and is formed by denting from the top end to the bottom end of the second housing 112.

In some other optional embodiments, the mounting port 1121 may alternatively be in a hole shape and penetrate the wall portion, at the top end side, of the second housing 112 in the thickness direction. The atomization seat 12 may be arranged in the hole-shaped mounting port 1121 through snapping, penetrating, etc.

The mounting port 1121 is provided, so that the atomization seat 12 is assembled to the housing 11 through the mounting port 1121 conveniently. Also, in a case that the housing 11 has the fixed mounting size in the thickness direction, the atomization seat 12 has a large mounting space, and the impact of the atomization seat 12 on the mounting size of the housing 11 in the thickness direction is reduced.

Specifically, in some examples, in the thickness direction of the housing 11, the side, farthest away from the first housing 111, of the atomization seat 12 is arranged in the mounting port 1121 in a penetrating manner and is flush with the outer surface of the second housing 112. In some other examples, the side, farthest away from the first housing 111, of the atomization seat 12 is arranged in the mounting port 1121 in a penetrating manner and is dented into the outer surface of the second housing 112. In yet some other examples, the side, farthest away from the first housing 111, of the atomization seat 12 is arranged in the mounting port 1121 in a penetrating manner and protrudes out of the outer surface of the second housing 112. In this way, when the housing 11 has the fixed size in the thickness direction, the size of the atomization seat 12 in the thickness direction can also have a plurality of options, so that the structural layout of the aerosol generating device is more proper.

The specific structure of the atomization seat 12 is not limited herein. Illustratively, with reference to FIG. 8, the atomization seat 12 includes a seat housing 121 and a raised post 122 protruding along the top surface of the seat housing 121 in the direction towards the top side. The seat housing 121 defines the atomization cavity 123. The raised post 122 penetrates out of the top end surface of the housing 11, and a space in the raised post 122 at least partially defines the air outlet channel 124.

In other words, the air outlet channel 124 is approximately formed at the top side of the atomization cavity 123. The aerosol generated in the atomization cavity 123 may be directly discharged through the raised post 122. The raised post 122 penetrates out of the top end surface of the housing 11. In other words, the raised post 122 is closer to the user, so that the aerosol can be conveyed from the air outlet channel 124 to the outside in short time, to be used by the user. The user may use the aerosol through a mouthpiece sleeving the raised post 122, etc. Certainly, the user may alternatively directly use the aerosol through the raised post 122.

In another aspect, the raised post 122 is approximately in a cylindrical shape, and thus is simple in structure and easy to manufacture. Thus, the structural requirement on the atomization seat 12 can be reduced, and the difficulty in manufacturing the atomization seat 12 can be reduced.

The raised post 122 may be fixedly or detachably connected to the atomization cavity 123. For example, in some embodiments, the raised post 122 and the atomization cavity 123 are integrally formed. In some other embodiments, the raised post 122 and the atomization cavity 123 are separate members and are connected through inserting, fastening, etc.

Illustratively, as further shown in FIG. 1 to FIG. 4, the aerosol generating device includes a filter 3. The filter 3 extends into and is fixed to the raised post 122, and communicates with the air outlet channel 124.

In the embodiment, the filter 3 is parallel to the axis of the housing 11 in the length direction.

Specifically, the aerosol generating substrate on the surface of or inside the portion, located in the atomization cavity 123, of the strip-shaped substrate 20 is heated and atomized to generate the aerosol, the aerosol flows to the filter 3 through the raised post 122, is then filtered by the filter 3, and is finally diffused outwards, to be used by the user. The filter 3 can adsorb large-particle atomized drops and a condensate that are generated by atomizing the aerosol generating substrate on the surface of or inside the strip-shaped substrate 20. Thus, the user is prevented from inhaling the large-particle atomized drops or the condensate, and the user experience is improved.

The filter 3 may be made of a soft material. For example, the filter 3 may be made of cellulose acetate, so that the filtering effect is desirable and the adsorption resistance is small.

Illustratively, with reference to FIG. 4 and FIG. 7, the housing assembly 10 further includes a partition member 13. One end of the partition member 13 is arranged at the inner surface of the first housing 111, and the other end of the partition member extends in the thickness direction. To be specific, the end, farther away from the inner surface of the first housing 111, of the partition member 13 extends towards the inner surface of the second housing 112 in the thickness direction.

The first housing 111 and the second housing 112 are in butt joint in the thickness direction and jointly define the storage space 101 and the accommodation space 102 along with the partition member 13. To be specific, the partition member 13 partitions the housing space 100 defined by the housing 11 into the storage space 101 and the accommodation space 102, and the partition member 13 at least partially forms the side wall of the storage space 101 and the side wall of the accommodation space 102.

Specifically, the partition member 13 is approximately in an S shape in the embodiment. Thus, the first reel 311 and the second reel 321 are located in two recesses of the S shape respectively, and the housing space 100 is more effectively utilized.

In some other optional embodiments, the partition member 13 may alternatively be in a circular shape, a C shape, an irregularly-cambered shape, a water drop shape, a rectangular shape, etc. The housing space 100 may be partitioned by the partition member into the storage space 101 and the accommodation space 102 for arranging the first reel 311 and the second reel 321 respectively.

In yet some other optional embodiments, the partition member 13 may alternatively be arranged on the second housing 112.

In the related art, a non-atomized aerosol generating substrate strip and an atomized aerosol generating substrate strip are mixed together, and thus spatial isolation cannot be achieved. Without the isolation design of the new aerosol generating substrate strip and the old aerosol generating substrate strip during atomization and storage, the new aerosol generating substrate strip and the old aerosol generating substrate strip are mixed together during atomization, and thus off-odor, etc. are caused to affect the user experience. The atomized aerosol generating substrate strip inevitably generates scraps. When the scraps are attached to the new aerosol generating substrate strip, the burnt odor is generated during the atomization.

In the atomization substrate assembly according to the embodiments of the present disclosure, the storage space 101 is spaced from the accommodation space 102. In other words, the non-atomized strip-shaped substrate 20 and the atomized strip-shaped substrate 20 are arranged in two independent spaces respectively, to isolate the non-atomized strip-shaped substrate 20 from the atomized strip-shaped substrate 20. The off-odor between the non-atomized strip-shaped substrate 20 and the atomized strip-shaped substrate 20 can be alleviated to a particular extent. In addition, the scraps generated by the atomized strip-shaped substrate 20 can also be prevented from being attached to the non-atomized strip-shaped substrate 20, so that the burnt odor can be avoided to a particular extent.

In the related art, the aerosol generating substrate strip is deformed, hardened, burred, and layered after atomized through the atomization assembly. However, the aerosol generating device in the related art is not specifically used for accommodating the atomized aerosol generating substrate strip, which probably further causes the atomized aerosol generating substrate strip to generate the scraps.

In the atomization substrate assembly according to the embodiments of the present disclosure, the housing assembly 10 is internally provided with the accommodation space 102 specially used for accommodating the atomized strip-shaped substrate 20, so that generation of the scraps can be reduced to a particular extent. The storage space 101 is spaced from the accommodation space 102, so that fewer scraps can enter the storage space 101 to be attached to the non-atomized strip-shaped substrate 20. In addition, the second reel 321 is arranged in the accommodation space 102 and used for winding the atomized strip-shaped substrate 20. By winding the atomized strip-shaped substrate 20 into a coil, the generation of the scraps can be further reduced. Moreover, by arranging the storage space 101 specially used for storing the non-atomized strip-shaped substrate 20 in the housing assembly 10, the non-atomized strip-shaped substrate 20 can be protected, so that the user experience is better.

Specifically, as shown in FIG. 4, the partition member 13 is in the shape of an elongated S-shaped plate. One end of the partition member is connected to the side wall of the atomization cavity 123, and the other end of the partition member is connected to the side wall of the first housing 111.

In some embodiments, the front end of the partition member 13 at least forming the storage space 101 is in sealing fit with the second housing 112. In this way, when the first housing 111 and the second housing 112 are in butt joint in the thickness direction, the front end of the partition member 13 forming the storage space 101 is in sealing fit with the second housing 112. To be specific, the storage space 101 is relatively sealed, so that the portion, in the storage space 101, of the strip-shaped substrate 20 can be effectively prevented from making contact with external air and becoming moist to a particular extent.

The front end of the partition member 13 indicates the end, closer to the front side, of the partition member 13, i.e. the end, closer to the second housing 112, of the partition member 13.

It should be noted that the front end of the partition member 13 at least forming the storage space 101 is in sealing fit with the second housing 112. It may indicate that the front end of only the partition member 13 forming the storage space 101 is in sealing fit with the second housing 112. In this case, at least the storage space 101 is relatively sealed. Alternatively, the front ends of all the partition members 13 may be in sealing fit with the second housing 112. In this case, the storage space 101 and the accommodation space 102 are relatively sealed.

In the related art, in a process of disassembling the package for use, or in a process of placement in a humid environment, the strip-shaped substrate is likely to become moist after adsorbing water. Thus, the strip-shaped substrate is mildewed or is undesirable in mouth feel, and the user experience is undesirable.

In the atomization substrate assembly 1 according to the embodiments of the present disclosure, the front end of the partition member 13 at least forming the storage space 101 is in sealing fit with the second housing 112. In this way, at least the storage space 101 is relatively sealed. In a process of disassembling the package for use, external air can be effectively prevented from entering the storage space 101 to a particular extent, so that the portion, in the storage space 101, of the strip-shaped substrate 20 is prevented from making contact with the external air and becoming moist. Thus, the strip-shaped substrate 20 remains in a fresh state, the service life of the strip-shaped substrate 20 is longer, the safety and the mouth feel consistency are higher, and the user experience is better.

The specific method for sealing fit between the front end of the partition member 13 and the second housing 112 is not limited herein. Illustratively, in some implementations, a sealing member may be arranged at the front end of the partition member 13, or a sealing member or a sealing portion (such as a recess adapted to the partition member 13) may be arranged at the position, corresponding to the partition member 13, of the second housing 112.

In some embodiments, when the storage space 101 and the accommodation space 102 can be partitioned only by causing the partition member 13 to be in contact with the side wall of the first housing 111, a sealing member may alternatively be arranged on the portion, in contact with the side wall of the first housing 111, of the partition member 13, to further improve the relative sealing performance of at least the storage space 101.

In some other optional embodiments, no sealing member is arranged between the partition member 13 and the side wall of the first housing 111. The partition member 13 is fixedly connected to the side wall of the first housing 111 through soldering, gluing, integral forming, etc., so that the sealing effect at the joint is achieved.

In yet some other optional embodiments, a sealing member may alternatively be arranged between the first housing 111 and the second housing 112, to prevent ingress of moisture, etc. along a gap at the joint between the first housing and the second housing after the first housing 111 and the second housing 112 are assembled into a whole.

It should be understood that the sealing member may be a common sealing structure such as a sealing pad made of rubber.

It should be noted that various specific atomization methods of the atomization assembly 220 are available. For example, the atomization is performed through heating. The heating includes, but is not limited to, resistance heating, electromagnetic heating, infrared heating, microwave heating, laser heating, etc. Certainly, the atomization may alternatively be performed through ultrasound, which is not specifically limited herein. The embodiment is described with the atomization assembly 220 employing the resistance heating as an example.

It should be noted that the specific position for arranging the atomization assembly 220 is not limited herein. Illustratively, in some embodiments, with reference to FIG. 2 and FIG. 3, the atomization assembly 220 is arranged at the main machine 2, and the atomization assembly 220 at least partially extends into the atomization cavity 123 to be used for atomizing the strip-shaped substrate 20. In other words, the atomization assembly 220 is directly arranged on the main machine 2, and to be specific, the power supply assembly and the atomization assembly 220 are arranged on the main machine 2, so that the reliability of electrical connection between the atomization assembly 220 and the power supply assembly can be improved. In addition, the atomization assembly 220 may be used along with the main machine 2 all the time, without being replaced along with the replacement of the atomization substrate assembly 1. Thus, the repeated use rate of the atomization assembly 220 is increased, and the use cost of the user is reduced.

Specifically, with reference to FIG. 5, the atomization assembly 220 includes the heating member 221. The heating member 221 is arranged on the housing assembly 10, extends into the atomization cavity 123, and is in at least partial contact with the strip-shaped substrate 20. The heating member 221 generates heat after energized, to heat the strip-shaped substrate 20.

Illustratively, with reference to FIG. 8, the side wall of the side, in the thickness direction of the atomization substrate assembly 1, of the atomization cavity 123 is provided with a first avoiding port 1211. The portion, corresponding to the first avoiding port 1211, of the housing 11 is provided with a second avoiding port 1111. The second avoiding port 1111 is in butt joint with the first avoiding port 1211. The first avoiding port 1211 is provided, so that the heating member 221 enters the atomization cavity 123 through the first avoiding port 1211 to be attached to the strip-shaped substrate 20, so as to heat and atomize the strip-shaped substrate 20. The second avoiding port 1111 is provided to facilitate the electrical connection and the signal connection between the power supply assembly of the main machine 2 and the atomization assembly 220.

Specifically, as further shown in FIG. 8, the side wall of the side, in the thickness direction of the atomization substrate assembly 1, of the seat housing 121 is provided with a first avoiding port 1211, and the portion, corresponding to the first avoiding port 1211, of the first housing 111 is provided with a second avoiding port 1111.

The shape of the heating member 221 is not limited, and the heating member may be in a cylindrical shape or a sheet shape. In some examples, the heating member 221 is formed into the sheet shape, to have a sufficiently large contact area with the portion, located in the atomization cavity 123, of the strip-shaped substrate 20. Thus, the atomization efficiency of the aerosol generating device is improved.

Understandably, the heating member 221 may be made of metal or ceramics, to heat the aerosol generating substrate for atomization through heat convection, heat conduction, etc.

In some other optional embodiments, the heating member 221 may alternatively be spaced from the strip-shaped substrate 20 (for example, when the atomization assembly 220 heats the strip-shaped substrate 20 through the infrared rays/electromagnetic waves/optical waves).

In some embodiments, the heating member 221 extends into the atomization cavity 123 to correspond to the portion, located in the atomization cavity 123, of the strip-shaped substrate 20 in the width direction of the strip-shaped substrate. Thus, the uniformity and symmetry of a temperature field in the thickness direction of the atomization cavity 123 are ensured, the heating effect of the strip-shaped substrate 20 is improved, and the mouth feel of inhalation is enhanced.

It should be understood that the "width" herein indicates the width of the strip-shaped substrate 20.

In some embodiments, the heating member 221 is centrally and symmetrically arranged in the atomization cavity 123 with respect to the axis of the atomization cavity 123 on the section perpendicular to the thickness direction, to further ensure the uniformity and symmetry of the temperature field in the atomization cavity 123.

In some embodiments, as further shown in FIG. 5, the atomization assembly 220 further includes a connection plate 222. The connection plate 222 is connected to the heating member 221. The connection plate 222 closes the second avoiding port 1111 or the first avoiding port 1211. The connection plate 222 is arranged to facilitate fixing of the heating member 221 and may be used for closing the second avoiding port 1111 or the first avoiding port 1211. Thus, the sealing performance and reliability of the atomization substrate assembly 1 are improved.

Specifically, the connection plate 222 is arranged at the second avoiding port 1111 of the first housing 111 in a covering manner, and closes the second avoiding port 1111. Thus, damage of debris entering the housing assembly 10 through the second avoiding port 1111 to the heating member 221 or the strip-shaped substrate 20 can be avoided to a particular extent. Certainly, in some other optional embodiments, the connection plate 222 may alternatively be arranged at the first avoiding port 1211 of the seat housing 121 in a covering manner and close the first avoiding port 1211.

Various connection methods for the connection plate 222 are available. Illustratively, in some embodiments, the connection plate 222 may be detachably connected to the housing 11 or the side wall of the atomization cavity 123. In this way, the atomization assembly 220 is replaced conveniently after the atomization substrate assembly 1 is used for a period of time or the atomization assembly 220 is damaged. For example, the connection plate 222 may be connected to the first housing 111 or the seat housing 121 through gluing, snapping, screwing, inserting, etc.

In some other embodiments, the connection plate 222 may alternatively be sandwiched between the first housing 111 and the second housing 112, to fix the connection plate 222. The connection plate 222 may alternatively be sandwiched between the first housing 111 and the seat housing 121, to fix the connection plate 222.

Illustratively, the atomization assembly 220 includes a connector 223 electrically connected to the heating member 221. The connector 223 is arranged at the side, facing away from the atomization cavity 123, of the connection plate 222. The connector 223 is used for being electrically connected to the power supply assembly. In other words, the atomization assembly 220 includes the heating member 221, the connection plate 222, and the connector 223. The connection plate 222 is used for connecting the heating member 221 to the connector 223, the heating member 221 and the connector 223 are arranged at two opposite sides of the connector 223 respectively, and the heating member 221 is electrically connected to a power supply through the connector 223.

Illustratively, in some embodiments, the connection plate 222 may be electrically connected to the heating member 221 and the connector 223. The heating member 221 may abut against the connector 223 or not. To be specific, the heating member 221 may be electrically connected to the connector 223 through the connection plate 222. In this case, the connection plate 222 is not only used for mounting the heating member 221 and the connector 223, but is also used for implementing the electrical connection between the heating member 221 and the connector 223. Certainly, in some other embodiments, the connection plate 222 may alternatively not be electrically connected to the heating member 221 and the connector 223, and the heating member 221 directly abuts against the connector 223 for the electrical connection. In this case, the connection plate 222 is only used for mounting the heating member 221 and the connector 223.

Certainly, in yet some other embodiments, the atomization assembly 220 may alternatively not be provided with the connector 223. When the atomization substrate assembly 1 is assembled to the main machine 2, the power supply assembly of the main machine 2 is electrically connected to the connection plate 222, so as to implement the electrical connection between the atomization assembly 220 and the power supply assembly.

In the embodiment, the atomization assembly 220 includes the connection plate 222, and the heating member 221 and the connector 223 that are arranged at the two opposite sides of the connection plate 222 respectively. When the atomization assembly 220 is assembled to the housing assembly 10, the heating member 221 extends into the atomization cavity 123 to be attached to the strip-shaped substrate 20. The connection plate 222 is arranged at the second avoiding port 1111 of the first housing 111 in a covering manner and closes the second avoiding port 1111. The connector 223 is arranged at the side, facing away from the atomization cavity 123, of the connection plate 222. The connector 223 penetrates out of the side surface of the first housing 111 in the thickness direction through the second avoiding port 1111 and is used for being electrically connected to the power supply.

Illustratively, with reference to FIG. 7, the feeding assembly 30 includes a storage disk 31 provided with the first reel 311, the storage disk 31 is rotatably arranged in the storage space 101, and the non-atomized strip-shaped substrate 20 is arranged around the storage disk 31. In other words, by arranging the storage disk 31, the storage disk 31 may rotate to facilitate unwinding of the strip-shaped substrate 20 from the storage disk 31. Thus, friction between the strip-shaped substrate 20 and the housing assembly 10 during unwinding is reduced.

Certainly, in some other embodiments, the feeding assembly 30 may alternatively not be provided with the storage disk 31, and the non-atomized strip-shaped substrate 20 is directly arranged on the housing assembly 10.

Illustratively, the feeding assembly 30 includes an accommodation disk 32 provided with the second reel 321. The accommodation disk 32 is rotatably arranged in the accommodation space 102, and the atomized strip-shaped substrate 20 is arranged around the accommodation disk 32. In other words, by arranging the accommodation disk 32, the accommodation disk 32 may rotate to facilitate winding of the strip-shaped substrate 20 around the accommodation disk 32. Thus, friction between the strip-shaped substrate 20 and the housing assembly 10 during winding is reduced.

Certainly, in some other embodiments, the feeding assembly 30 may alternatively not be provided with the accommodation disk 32, and the atomized strip-shaped substrate 20 is directly arranged on the housing assembly 10.

In the embodiment, the storage disk 31 includes the first reel 311 and two disk-shaped portions that are oppositely arranged at two ends of the first reel 311 in parallel. The accommodation disk 32 includes the second reel 321 and two disk-shaped portions that are oppositely arranged at two ends of the second reel 321 in parallel. The storage disk 31 and the accommodation disk 32 are arranged, so that the strip-shaped substrate 20 can be accommodated, and the strip-shaped substrate 20 can also be prevented from be separated from the first reel 311 or the second reel 321.

After atomized through the atomization assembly 220, the strip-shaped substrate 20 may be deformed, hardened, burred, and layered. In a natural state, the size of the strip-shaped substrate 20 is increased. In other words, for the strip-shaped substrate having the same length, the diameter of the non-atomized strip-shaped substrate wound into a coil is less than that of the atomized strip-shaped substrate wound into a coil. Thus, a space required for accommodating the atomized strip-shaped substrate 20 is greater than that required for storing the non-atomized strip-shaped substrate 20. Illustratively, on a plane perpendicular to the thickness direction of the housing assembly 10, the cross-sectional area of the accommodation space 102 is greater than that of the storage space 101. In this way, the accommodation space 102 is bigger than the storage space 101.

Illustratively, on a plane perpendicular to the thickness direction of the housing assembly 10, the cross-sectional area of the accommodation disk 32 is greater than that of the storage disk 31. In this way, the accommodation space 102 is bigger than the storage space 101.

In the embodiment, the size of the disk-shaped portions of the storage disk 31 is identical to the size of the disk-shaped portions of the accommodation disk 32. In some other optional embodiments, the size of the disk-shaped portions of the accommodation disk 32 may be greater than that of the disk-shaped portions of the storage disk 31, to accommodate the atomized strip-shaped substrate 20 increased in size.

In yet some other optional embodiments, the size of the disk-shaped portions of the accommodation disk 32 may alternatively be less than that of the disk-shaped portions of the storage disk 31.

Understandably, to improve the atomization effect of the strip-shaped substrate 20, the strip-shaped substrate 20 needs to be in close contact with the heating member 221 of the atomization assembly 220. The storage space 101 is arranged at the position farther away from the atomization cavity 123, so that the traveling distance for the non-atomized strip-shaped substrate 20 in the storage space 101 to enter the atomization cavity 123 after unwinding can be relatively extended, and the traveling trajectory of the non-atomized strip-shaped substrate 20 can be adjusted. Thus, the strip-shaped substrate 20 travels more flatly to be in close contact with the heating member, and damage to the non-atomized strip-shaped substrate 20 and the heating member is avoided. In addition, the accommodation space 102 is arranged at the position closer to the atomization cavity 123, so that the traveling distance for the atomized strip-shaped substrate 20 to enter the accommodation space 102 for winding can be relatively shortened, and generation of the scraps of the atomized strip-shaped substrate 20 can be reduced to a particular extent.

As shown in FIG. 7, in some embodiments, the atomization substrate assembly 1 further includes a guide assembly 40. The guide assembly 40 includes at least one guide member. The guide member is arranged in the housing space 100 and used for guiding the strip-shaped substrate 20, i.e. guiding the non-atomized portion, around the first reel 311, of the strip-shaped substrate 20 into the atomization cavity 123 and then to the second reel 321 through the atomization cavity 123. Thus, the movement route of the strip-shaped substrate 20 in the housing space 100 is planned.

Specifically, the guide assembly 40 may include at least two guide members. The strip-shaped substrate 20 is guided from the first reel 311 to the second reel 321 through the two guide members. The portion, between the two guide members, of the strip-shaped substrate 20 is arranged in the atomization cavity 123 in a penetrating manner. At least two guide members are arranged, so that the movement path of the flexible strip-shaped substrate 20 in the housing space 100 can be fixed, the flexible strip-shaped substrate extends along the smooth route, and damage of bending at a small angle to the strip-shaped substrate 20 is avoided. Moreover, it is ensured that the portion, in the atomization cavity 123, of the strip-shaped substrate 20 may be attached to the heating member 221 or arranged in parallel to the heating member. Moreover, the strip-shaped substrate 20 is protected, bending of the strip-shaped substrate 20 generated when the strip-shaped substrate enters or exists the atomization cavity 123, makes contact with the heating member 221, leaves the heating member, or is stuck at another position is avoided.

As shown in FIG. 4, in the embodiment, each guide member of the guide assembly 40 is arranged on the inner wall surface of the first housing 111. Three guide members are provided, which are defined as a first guide member 41, a second guide member 42, and a third guide member 43 respectively. The first guide member 41 and the second guide member 42 are arranged in the storage space 101, and the third guide member 43 is arranged in the accommodation space 102. The second guide member 42 and the third guide member 43 are approximately located at the same position as the atomization cavity 123 in the top-bottom direction (the up-down direction), so that the portion, between the second guide member 42 and the third guide member 43, of the strip-shaped substrate 20 is linearly arranged in the atomization cavity 123 in a penetrating manner and attached to (or parallel to) the heating member 221 in the atomization cavity 123. The first guide member 41 is arranged between the second guide member 42 and the first reel 311, and an included angle α (α is greater than zero degree) exists between the extension direction of the portion, between the first reel 311 and the first guide member 41, of the strip-shaped substrate 20 and the extension direction of the portion, between the first guide member 41 and the second guide member 42, of the strip-shaped substrate 20.

The first guide member 41 is arranged to cooperate with the S-shaped partition member 13. Thus, damage of contact friction over a large area to the strip-shaped substrate 20 because the strip-shaped substrate output from the first reel 311 touches the back surface of the recess portion, where the second reel 321 is located, of the S-shaped partition member 13 between reaching the second guide member 42 is avoided.

Specifically, in the embodiment, the extension direction of the portion, between the first guide member 41 and the second guide member 42, of the strip-shaped substrate 20 is approximately perpendicular to the extension direction of the portion, between the second guide member 42 and the third guide member 43, of the strip-shaped substrate 20, so that the portion, between the first reel 311 and the second reel 321, of the strip-shaped substrate 20 is distributed along the side profile of the housing space 100 to the greatest extent. Thus, distribution of the members in the housing space 100 is optimized, and space waste caused by partitioning the housing space 100 into a plurality of smaller spaces in the traveling direction of the strip-shaped substrate 20 is avoided.

In some other optional embodiments, the extension direction of the portion, between the first guide member 41 and the second guide member 42, of the strip-shaped substrate 20 may alternatively not be perpendicular to the extension direction of the portion, between the second guide member 42 and the third guide member 43, of the strip-shaped substrate 20. The guide assembly 40 may alternatively not be provided with the first guide member 41.

In yet some other optional embodiments, one, two, four, five, or more guide members may alternatively be provided.

In some embodiments, each guide member of the guide assembly 40 is in a cylindrical shape. The guide member may be a polished post fixed to the housing 11 and is only used for guiding. The strip-shaped substrate 20 is in friction contact with the guide member during transmission.

In some other optional embodiments, the guide member may alternatively be a rotary post rotatable along the axis of the guide member. During transmission of the strip-shaped substrate 20, no friction force is generated between the strip-shaped substrate 20 and the guide member, and the guide member conveys the strip-shaped substrate 20 through rotation of the guide member. The guide member may partially be a polished post fixed to the housing 11 and partially be a rotary post.

In the embodiment, each guide member of the guide assembly 40 is in a cylindrical shape.

In some other optional embodiments, the guide member may alternatively be in a polygonal post shape, an elliptic post shape, etc. When the guide member is arranged in a polygonal shape, rounded corners are arranged between the adjacent side end surfaces of the guide member, to avoid damaging the strip-shaped substrate 20.

In yet some other optional embodiments, the guide member may alternatively be in a shape having the cambered section in the thickness direction and is fixed to the first housing 111, with the cambered portion of the guide member facing away from the strip-shaped substrate 20.

In some embodiments, each guide member of the guide assembly 40 may alternatively be provided with a limiting structure for preventing separation. For example, a limiting plate is arranged at the end portion, farthest away from the first housing 111, of the guide member, so that the section of the guide member is approximately T-shaped, to prevent the strip-shaped substrate 20 from being separated.

In some other optional embodiments, the limiting structure may alternatively not be arranged on the guide member. The guide member is arranged in a cylindrical shape. The position, corresponding to the guide member, of the limiting structure is arranged on the second housing 112. After the second housing 112 is assembled to the first housing 111, the limiting structure is located between the second housing 112 and the end portion of the guide member, so that the strip-shaped substrate 20 can also be prevented from being separated. Moreover, the machining technology of the guide member can be simplified, and the manufacturing cost of the guide member can be reduced.

In yet some other optional embodiments, no limiting structure is provided. The height of the guide member is changed, so that after the first housing 111 is assembled to the second housing 112, two ends of the guide member abut with the first housing 111 and the second housing 112 respectively. In this case, the second housing 112 can also functions as the limiting structure to prevent separation.

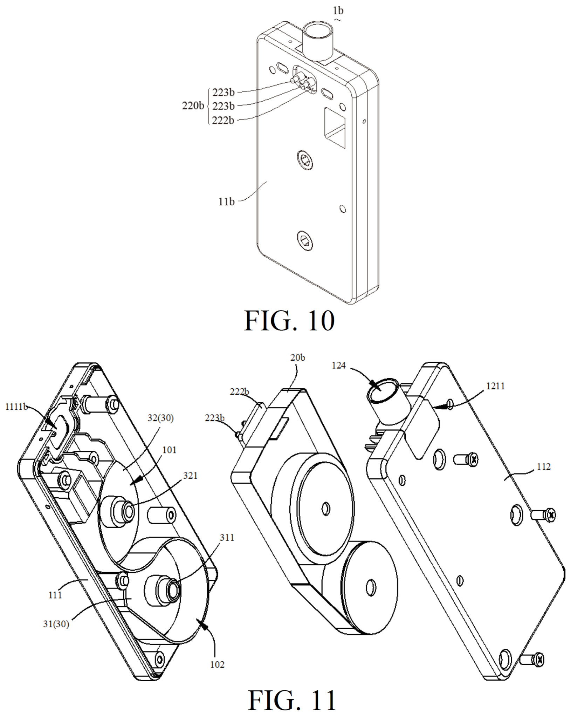

FIG. 9 to FIG. 11 show an aerosol generating device in a second embodiment of the present disclosure. The differences from the aerosol generating device in the first embodiment are as follows:
As shown in FIG. 10, in the embodiment, an atomization substrate assembly 1b includes an atomization assembly 220b. The atomization assembly 220b is arranged on a housing assembly 10b. The atomization assembly 220b at least partially extends into an atomization cavity (not shown in the figure) and is used for atomizing a strip-shaped substrate 20b. In other words, the atomization assembly 220b is connected to the housing assembly 10b. When a user disassembles the atomization substrate assembly 1b, the relative position of the housing assembly 10b and the atomization assembly 220b is not changed.

Understandably, to ensure the atomization efficiency and reliability of the atomization assembly 220b, the strip-shaped substrate 20b needs to be in close contact with the atomization assembly 220b. To be specific, the strip-shaped substrate 20b is completely attached to the atomization assembly 220b, so that the atomization assembly 220b atomizes the strip-shaped substrate 20b through heating. If a gap exists between the strip-shaped substrate 20b and the atomization assembly 220b, dry heating of the atomization assembly 220b is caused, and further, the atomization efficiency and reliability of the atomization assembly 220b are reduced. To resolve the above problem, in the embodiment of the present disclosure, the atomization assembly 220b is arranged at the housing assembly 10b. In this way, when the user disassembles the atomization substrate assembly 1b, the relative position of the housing assembly 10b and the atomization assembly 220b is not changed, so that the impact of a mounting gap and mounting instability of the user on the assembly reliability of the atomization assembly 220b and the strip-shaped substrate 20b can be reduced to a particular extent. The position between the strip-shaped substrate 20b and the atomization assembly 220b is not changed after replacement with a new strip-shaped substrate 20b. To be specific, the strip-shaped substrate 20b can be completely attached to the atomization assembly 220b. In addition, the atomization assembly 220b is arranged at the housing assembly 10b. When the replacement with the new strip-shaped substrate 20b is performed, the strip-shaped substrate 20b can be directly and completely attached to the atomization assembly 220b, so as to prevent incorrect mounting, further improving the reliability of the atomization assembly 220b.

In the embodiment, the atomization assembly 220b is separately connected to an atomization seat 12b and a housing 11b. A connection plate 222b of the atomization assembly is connected to the housing 11b. A heating member extends into an atomization cavity defined by the atomization seat 12b. Two connections 223b extend out of the housing 11b through a second avoiding port 1111b.

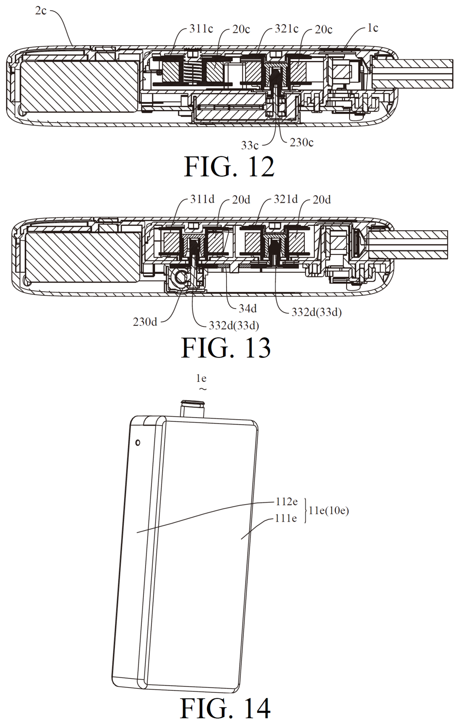

FIG. 12 shows an aerosol generating device in a third embodiment of the present disclosure. The differences from the aerosol generating device in the first embodiment are as follows:
In the embodiment, one driving assembly 230c is provided, partially extends into an atomization substrate assembly 1c, and is connected to a second reel 321c, so as to drive the second reel 321c to rotate.

The second reel 321c is used for accommodating an atomized strip-shaped substrate 20c. Thus, in a process of driving the second reel 321c to rotate, the strip-shaped substrate 20c wrapped around a first reel 311c and the second reel 321c pulls the first reel 311c to synchronously rotate, so that the new non-atomized portion of the strip-shaped substrate 20c is released to be atomized.

When to inhale an aerosol, the user only needs to drive the second reel 321c. The second reel 321c rotates under driving by the driving assembly 230c, to drive the first reel 311c to synchronously rotate, so that the strip-shaped substrate 20c is continuously atomized. Thus, a number of the driving assembly 230c arranged and the cost are reduced.

One connection structure 33c is provided in the embodiment and arranged in correspondence to the second reel 321c.

Illustratively, the first reel 311c is a damping rotary shaft. To be specific, the first reel 311c undergoes the damping force during rotation. For example, the first reel 311c is connected to a damping gear, an electric motor, or a torsional spring, or is in interference fit with either one of the atomization substrate assembly 1c and a main machine 2c.

The first reel 311c is arranged to undergo the damping force during rotation, so that the strip-shaped substrate 20c has the pre-tightened force during rotation. Thus, the reliability of the strip-shaped substrate 20c during movement is improved, and damage to the strip-shaped substrate 20c during the movement is avoided.

FIG. 13 shows an aerosol generating device in a fourth embodiment of the present disclosure. The differences from the aerosol generating device in the third embodiment are as follows:
In the embodiment, two connection structures 33d are provided. The two connection structures 33d are arranged in correspondence to the first reel 311d and the second reel 321d respectively. A feeding assembly further includes a transmission member 34d. The transmission member 34d is connected to connection members 332d of the two connection structures 33d separately.

A driving assembly 230d drives the second reel 321d to rotate through the connection structure 33d. The connection member 332d of the connection structure 33d is driven by the driving assembly 230d to rotate, and the connection member 332d drives the connection member 332d of the other connection structure 33d to rotate through the transmission member 34d simultaneously. Thus, one driving assembly 230d simultaneously drives the first reel 311d and the second rotary shaft 32d to synchronously rotate.

The transmission member 34d is arranged to avoid the pulling force probably caused by the first reel 311d on the strip-shaped substrate 20d when the drive force is not received. Thus, the stress on the strip-shaped substrate 20d during transmission is reduced, and the strip-shaped substrate is protected. Moreover, it is not required to arrange two driving assemblies 230d. Thus, a number of members of the aerosol generating device is reduced, and the production cost is reduced.

Specifically, the transmission member 34d may be a transmission gear. The transmission gear may be arranged at the side, farthest away from the first reel 311d and the second reel 321d, of the connection member 332d. Thus, one driving assembly 230d drives the first reel 311d and the second reel 321d to synchronously rotate through the transmission connection among three gears.

In some other optional embodiments, the connection member 34d may alternatively be a rotary member. A transmission belt is arranged between the connection member 34d and each of the two connection members 332d, to achieve synchronous rotation of the first reel 311d and the second reel 321d.

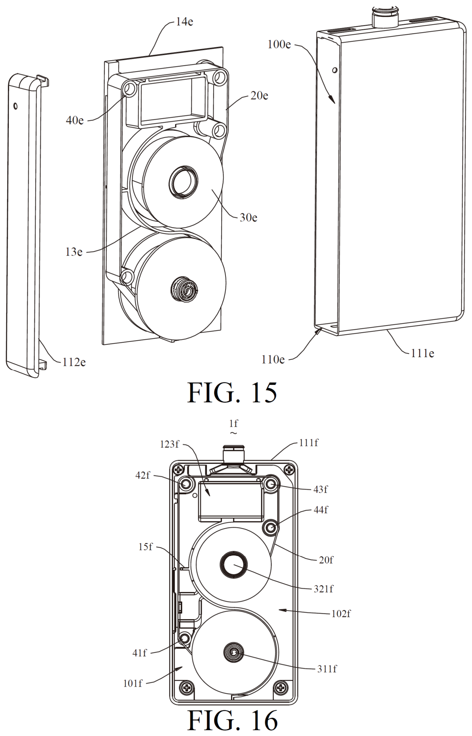

FIG. 14 and FIG. 15 show an atomization substrate assembly 1e in a fifth embodiment of the present disclosure. The differences from the atomization substrate assembly 1 in the first embodiment are as follows:
In the embodiment, a housing assembly 10e further includes a mounting plate 14e. A strip-shaped substrate 20e, a feeding assembly 30e, a guide assembly 40e, and a partition member 13e are arranged on the mounting plate 14e. The mounting plate 14e is removably placed in a housing 11e. In this way, when the strip-shaped substrate 20e is to be replaced during use, only a first housing 111e and a second housing 112e need to be disassembled from each other, and the mounting plate 14e and all members on the mounting plate are replaced as a whole. Thus, a user does not need to mount a new strip-shaped substrate 20e on the feeding assembly 30e, and the user experience is improved. Alternatively, the user does not need to replace an entire atomization substrate assembly 1e, so that the use cost of the user is reduced.

Further, in the embodiment, the first housing 111e is in a shape of a hollow square body. An interior of the first housing is a housing space 100e. An opening 110e is formed in one side of the first housing, so that the housing space 100e can communicate with the outside. The second housing 112 is in a rectangular shape, has the size adapted to the size of the opening 110e, and is detachably arranged on the opening 110e, so that the housing space 100e is closed. The mounting plate 14e is in a rectangular shape, and has the height adapted to the height of the opening 110e and the size adapted to the size of the housing space 100e. Thus, the mounting plate can enter or exit the housing space 100e through the opening 110e.

In some other optional embodiments, the first housing 111e may alternatively be in a hollow polygonal shape, elliptical shape, elongated runway shape, etc. The mounting plate 14e may alternatively be in another shape correspondingly.

In the embodiment, the opening 110e is located in the left side of the first housing 111e. The upper end portion and the lower end portion of the second housing 112e are provided with fastening structures, and the second housing may be detachably connected to the first housing 111e through the fastening structures.

In some other optional embodiments, the opening 110e may alternatively be located in the right side, the top side, the bottom side, etc. of the first housing 111e. The first housing 111e may alternatively be rotatably connected to the second housing 112e.

It should be understood that the method for the rotatable connection between the first housing 111e and the second housing 112e is not limited herein. The rotatable connection may be implemented through a hinge structure, a rotary connection shaft, an integrally-formed flexible connection portion, etc.

FIG. 16 shows an atomization substrate assembly 1f in a sixth embodiment of the present disclosure. The differences from the atomization substrate assembly 1 in the first embodiment are as follows:
In the embodiment, a guide assembly further includes a fourth guide member 44f. The fourth guide member 44f is arranged in an accommodation space 102f and arranged between a third guide member 43f and a second reel 321f. Thus, the angle change of the extension direction of a strip-shaped substrate 20f at the third guide member 43f (an increase in the included angle between the portion, between a second guide member 42f and the third guide member 43f, of the strip-shaped substrate 20f and the portion, at the other side of the third guide member 43f, of the strip-shaped substrate 20f) is reduced. Further, the contact area between the strip-shaped substrate 20f and the third guide member 43f can be narrowed, so that extension of the strip-shaped substrate 20f is smoother, and further, a friction force borne by the strip-shaped substrate 20f during conveying can be reduced.

Further, the extension direction of the portion, between the fourth guide member 44f and the third guide member 43f, of the strip-shaped substrate 20f is parallel to the extension direction of the portion, between a first guide member 41f and the second guide member 42f, of the strip-shaped substrate 20f, and is perpendicular to the extension direction of the portion, between the second guide member 42f and the third guide member 43f, of the strip-shaped substrate 20f. Thus, the portion, between the first guide member 41f and the fourth guide member 44f, of the strip-shaped substrate 20f approximately extends along the left side wall, the top side wall, and the right side wall of the first housing 111f, so that a smaller space is occupied.

Further, at least one stop position 15f is further formed between the first guide member 41f and the second guide member 42f. A channel for the strip-shaped substrate 20f to extend is formed at the stop position 15f, to divide a storage space 101f into several smaller spaces. Thus, it is further ensured that the portion, around the first reel 311f, of the strip-shaped substrate 20f is not polluted by the atomized portion of the strip-shaped substrate 20f.

Specifically, in the embodiment, one stop position 15f is provided and formed by adding a lug plate to a back plate at a recess, corresponding to the second reel 321f, of the S-shape portion of a partition member 13f and adding a lug plate at the position corresponding to the first housing 111f. The channel is formed at an interval between two lug plates, and the width of the channel is adapted to the sectional thickness of the strip-shaped substrate 20f, so that extension of the strip-shaped substrate 20f is ensured.

The stop position 15f is arranged to form one separate cavity for transition between the atomization cavity 123f and a cavity where the first reel 311f is located. The size of the channel is adapted to the size of the strip-shaped substrate 20f. Equivalently, the size of a communication channel between the cavities at two sides of the stop position 15f is reduced, and a new isolation barrier is formed. Thus, the closure performance of the cavity where the first reel 311f is located can be further ensured, pollution to the non-atomized portion, around the first reel 311f, of the strip-shaped substrate 20f after an aerosol formed through atomization in the atomization cavity 123f directly permeates into the cavity where the first reel 311f is located through an inlet of the atomization cavity 123f can be avoided.

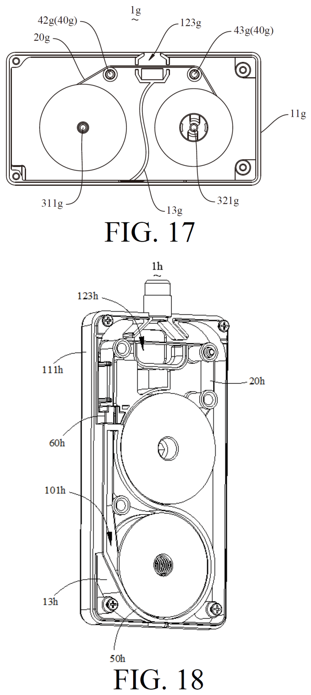

FIG. 17 shows an atomization substrate assembly 1g in a seventh embodiment of the present disclosure. The differences from the atomization substrate assembly 1 in the first embodiment are as follows:
In the embodiment, a filter (not shown in the figure) is at least partially perpendicular to the axis of a housing 11g in the length direction. An atomization cavity 123g is formed on the side wall of the housing 11g in the length direction. The atomization cavity 123g is located between a first reel 311g and a second reel 321g.

Further, two ends of a partition member 13g in the length direction are connected to the wall surface of the atomization cavity 123g and another side wall opposite the side wall where the atomization cavity 123g is located of the housing 11g in the length direction respectively.

In the embodiment, the partition member 13g is in a wave shape.

In some other optional embodiments, the partition member 13g may alternatively be in an S shape or a linear shape.

Further, only two guide members (including only a second guide member 42g and a third guide member 43g) of a guide assembly 40g are provided in the embodiment. The second guide member 42g is located between the first reel 311g and the atomization cavity 123g. The third guide member 43g is located between the second reel 321g and the atomization cavity 123g. The second guide member 42g and the third guide member 43g are located at two sides of the atomization cavity 123g respectively.

By changing the position relationship among the atomization cavity 123g, the first reel 311g, and the second reel 321g, a number of the guide members arranged can be reduced, and the production cost of the atomization substrate assembly 1g can be reduced. Moreover, the exposed length of the portion, between the first reel 311g and the second reel 321g, of the strip-shaped substrate 20g is shortened, and the length of the portion, between the first reel 311g and the atomization cavity 123g, of the strip-shaped substrate 20g is further shortened. Thus, the degree of pollution of an aerosol generated through atomization in the atomization cavity 123g to such a portion of the strip-shaped substrate 20g can be reduced, and the mouth feel of inhalation of a user is enhanced.

FIG. 18 to FIG. 20 show an atomization substrate assembly 1h in an eighth embodiment of the present disclosure. The differences from the atomization substrate assembly 1f in the sixth embodiment are as follows:
With reference to FIG. 18, the atomization substrate assembly 1h includes a first sealing member 50h. The first sealing member 50h is sandwiched between a second housing 112h and the front end of a partition member 13h defining a storage space 101h. To be specific, the first sealing member 50h is sandwiched between the front end of the side wall of the storage space 101h and the second housing 112h.

In other words, the first sealing member 50h is arranged and used for being sandwiched between the second housing 112h and the front end of the partition member 13h forming the storage space 101h. To be specific, the first sealing member 50h is used for sealing a gap between the front end of the side wall of the storage space 101h and the second housing 112h. Thus, external air can be prevented from entering the storage space 101h through the gap between the second housing 112h and the front end of the partition member 13h to a particular extent, and the portion, in the storage space 101h, of the strip-shaped substrate 20h can be prevented from making contact with the external air and becoming moist.

The first sealing member 50h is, for example, an elastic member elastically deformable. The first sealing member 50h is elastically deformed, to implement the sealing fit between the front end of the partition member 13h and the second housing 112h.

Specifically, the first sealing member 50h is, for example, a silicon pad or a rubber pad.

In some other implementations, the atomization substrate assembly 1h may alternatively not provided with the first sealing member 50h. Instead, the front end of the partition member 13h abuts against the inner surface of the second housing 112h, to seal the gap between the front end of the side wall of the storage space 101h and the second housing 112h. Thus, the front end of the partition member 13h is in sealing fit with the second housing 112h.

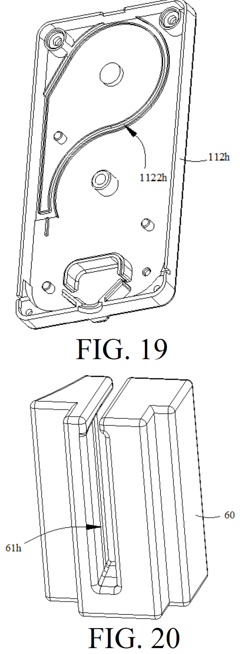

Illustratively, with reference to FIG. 19, the second housing 112h is provided with a sealing recess 1122h. The first sealing member 50h is arranged in the sealing recess 1122h. The second housing 112h is provided with the sealing recess 1122h. Thus, the first sealing member 50h is located conveniently, shifting of the first sealing member 50h is avoided, the assembly reliability of the first sealing member 50h is improved, and the reliability of a sealing structure between the partition member 13h and the second housing 112h is further improved.

It should be noted that the specific method for providing the sealing recess 1122h in the second housing 112h is not limited herein. Illustratively, in some implementations, with reference to FIG. 11, some regions of the second housing 112h may be dented to form the sealing recess 1122h. In some other implementations, a raised rib may be arranged on the second housing 112h, and the sealing recess 1122h is delimited by the raised rib.

In some other optional embodiments, the partition member 13h may alternatively be arranged on the second housing 112h, and the first sealing member 50h is arranged at the rear end (the end side closer to the first housing 111h) of the partition member 13h. Thus, sealing after the first housing 111h is assembled to the second housing 112h is achieved. The partition member 13h and the first sealing member 50h may alternatively be arranged on the first housing 111h and the second housing 112h respectively. The partition members 13h are alternatively arranged on the first housing 111h or the second housing 112h respectively. The sum of the thicknesses of the two partition members 13h is equal to the thickness generated after the first housing 111h is assembled to the second housing 112h. The first sealing member 50h is arranged between the two partition members 13h.

Illustratively, the side wall of the storage space 101h is provided with an outlet. The portion, in the storage space 101h, of the strip-shaped substrate 20h may move out of the storage space 101h through the outlet, and the portion, moved out of the storage space 101h through the outlet, of the strip-shaped substrate 20h enters the atomization cavity 123h to be heated and atomized. The specific position for providing the outlet is not limited herein. Illustratively, the outlet is provided in the top of the storage space 101h. In this way, the outlet may be arranged closer to the atomization cavity 123h, so that the distance between the storage space 101h and the atomization cavity 123h is shortened.

With reference to FIG. 3, FIG. 10, and FIG. 12, the atomization substrate assembly 1h includes a second sealing member 60h arranged at the outlet. The second sealing member 60h is used for sealing the outlet. To be specific, the second sealing member 60h is used for sealing a gap between the outlet and the strip-shaped substrate 20h. Thus, external air can be prevented from entering the storage space 101h through the gap between the outlet and the strip-shaped substrate 20h to a particular extent, and the portion, in the storage space 101h, of the strip-shaped substrate 20h can be prevented from making contact with the external air and becoming moist.

The strip-shaped substrate 20h and the second sealing member 60h are in sealing fit and movable relative to each other. In this way, the external air can be prevented from entering the storage space 101h through the gap between the strip-shaped substrate 20h and the second sealing member 60h to a particular extent, and the portion, in the storage space 101h, of the strip-shaped substrate 20h can be prevented from making contact with the external air and becoming moist. Also, the strip-shaped substrate 20h and the second sealing member 60h are movable relative to each other, so that the strip-shaped substrate 20h can smoothly enter the atomization cavity 123h from the storage space 101h.

Specifically, with reference to FIG. 18 and FIG. 20, the second sealing member 60h is provided with a through recess 61h. The strip-shaped substrate 20h is movable arranged in the through recess 61h in a penetrating manner and is in sealing fit with the recess wall of the through recess 61h. The second sealing member 60h is used for sealing the outlet. The second sealing member 60h is provided with the through recess 61h for the strip-shaped substrate 20h to pass through. Further, the strip-shaped substrate 20h is arranged to be in sealing fit with the recess wall of the through recess 61h, so that the sealing performance of the storage space 101h is further improved.

Illustratively, the second sealing member 60h is, for example, an elastic member elastically deformable. The second sealing member 60h is elastically deformed, so that the strip-shaped substrate 20h and the second sealing member 60h are in sealing fit and movable relative to each other.

Specifically, the second sealing member 60h is, for example, a silicon member or a rubber member. A mounting space is arranged in the first housing 111h, and the second sealing member 60h is arranged in the mounting space. The second sealing member 60h may alternatively employ the contour design. To be specific, the second sealing member 60h is arranged to match the mounting space in outline.

A limiting member is arranged in the first housing 111h and used for limiting the second sealing member 60h. Thus, shifting of the second sealing member 60h during movement of the strip-shaped substrate 20h can be avoided, and the reliability of the atomization substrate assembly 1h can be improved.

A comparison of weight gain percentages of moist samples after a moisture-proof test of the atomization substrate assembly 1h without the above sealing measure and the atomization substrate assembly 1h with the above sealing measure is as follows:
In a 6-hour moisture-proof test, the weight gain percentage of the atomization substrate assembly 1h without the above sealing measure is 0.181%, and the weight gain percentage of the atomization substrate assembly 1h with the above sealing measure is 0.107%.

In a 24-hour moisture-proof test, the weight gain percentage of the atomization substrate assembly 1h without the above sealing measure is 0.384%, and the weight gain percentage of the atomization substrate assembly 1h with the above sealing measure is 0.176%.

As can be seen, the atomization substrate assembly 1h with the above sealing measure has better sealing performance, so that the external air can be effectively prevented from entering the storage space 101h to a particular extent, and the portion, in the storage space 101h, of the strip-shaped substrate 20h can be prevented from making contact with the external air and becoming moist. Thus, the strip-shaped substrate 20h remains in a fresh state, the service life of the strip-shaped substrate 20h is longer, the safety and the mouth feel consistency are higher, and the user experience is better.

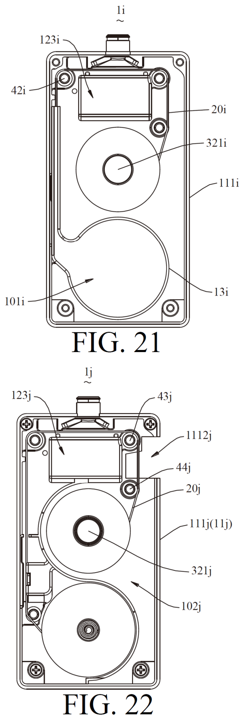

FIG. 21 shows an atomization substrate assembly 1i in a ninth embodiment of the present disclosure. The differences from the atomization substrate assembly 1f in the sixth embodiment are as follows:
In the embodiment, a partition member 13i is in a cover shape and includes the top wall and the side wall. The rear end of the side wall of the partition member is connected to a first housing 111i, and the front end of the side wall is connected to the top wall, to form a semi-closed structure. The partition member is fastened to a first reel (not shown in the figure) and defines a storage space 101i along with the first housing 111i without cooperating with a second housing (not shown in the figure). Thus, fewer sealing members are used, and no stop position is arranged. Moreover, the sealing effect inside the storage space 101i is ensured, and a non-atomized strip-shaped substrate 20i is protected against pollution.

Specifically, a first guide member (not shown in the figure) and at least the portion, between the first guide member and a second guide member 42i, of the strip-shaped substrate 20i are also accommodated in the cover-shaped partition member 13i. The side wall of the partition member 13i forms a lead-out port adapted to the section of the strip-shaped substrate 20i at the location of the portion, between the first guide member and the second guide member 42i, of the strip-shaped substrate 20i. The strip-shaped substrate 20i extends out of the storage space 101i through the lead-out port and enters the accommodation space 102i. Thus, the size of an opening that enables communication between the storage space 101i and the outside is further reduced, and the influence of an excessively large opening on the storage effect of the non-atomized strip-shaped substrate 20i is avoided.

In some embodiments, a sealing member may also be arranged at the lead-out port, to improve the closure effect on the storage space 101i.

In some other optional embodiments, the cover-shaped partition member 13i may alternatively be fastened to the second reel 321i, or the second reel 321i and the atomization cavity 123i are housed in the partition member. Thus, the non-atomized portion of the strip-shaped substrate 20i is isolated from the atomized portion of the strip-shaped substrate 20i, and the non-atomized portion of the strip-shaped substrate 20i is prevented from being polluted by atomized gas containing an aerosol, etc.

FIG. 22 shows an atomization substrate assembly 1j in a tenth embodiment of the present disclosure. The differences from the atomization substrate assembly 1f in the sixth embodiment are as follows:
In the embodiment, a pouring port 1112j is formed in a first housing 111j, to pour scraps, such as residuals generated by an atomized strip-shaped substrate 20j.

Specifically, the pouring port 1112j is formed in the portion, between a third guide member 43j and a fourth guide member 44j, of the first housing 111j and penetrates the rear side wall and the right side wall of the first housing 111j separately.

The portion, between a second guide member 42j and the third guide member 43j, of the strip-shaped substrate 20j is arranged in the atomization cavity 123j in a penetrating manner for atomization. Thus, the scraps probably generated by the atomized strip-shaped substrate 20j are separated from the strip-shaped substrate 20j. After the strip-shaped substrate 20j passes through the third guide member 43j, the extension direction is changed from the horizontal direction to the vertical direction, and the portion, between the third guide member 43j and the fourth guide member 44j, of the strip-shaped substrate 20j is closer to the right side wall of the first housing 111j. Thus, a channel at the position between the third guide member 43j and the fourth guide member 44j is narrow, and the scraps are likely to be accumulated. After accumulation for a long time, the strip-shaped substrate 20j is stuck, which is not conducive to conveying of the strip-shaped substrate 20j to a second reel 321j. The pouring port 1112j is arranged to pour the scraps out of a housing 11j in time, so as to avoid accumulation of the scraps in the accommodation space 102j (especially at the position between the third guide member 43j and the fourth guide member 44j). Thus, the service life of the atomization substrate assembly 1j is prolonged, and the user experience is enhanced.

In some other optional embodiments, the pouring port 1112j may alternatively penetrate only the rear side wall or the right side wall of the first housing 111j. The pouring port 1112j may alternatively be provided in the position corresponding to the second housing (not shown in the figure).

In some other optional embodiments, the pouring port 1112j may alternatively not be provided at the position between the third guide member 43j and the fourth guide member 44j. It should be understood that the pouring port may be provided in any position as long as the accommodation space 102j can communicate with the outside or the scraps separated from the atomized strip-shaped substrate 20j can be poured out of the housing 11j.

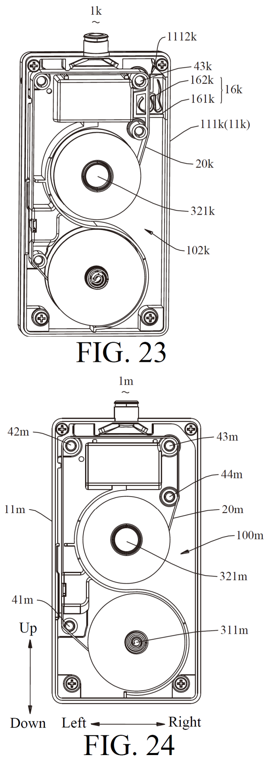

FIG. 23 shows an atomization substrate assembly 1k in an eleventh embodiment of the present disclosure. The differences from the atomization substrate assembly 1j in the tenth embodiment are as follows:
A housing assembly further includes an ash collection member 16k. The ash collection member 16k is arranged on a pouring port 1112k in a covering manner. Thus, during inhalation by a user, scraps generated through the atomization substrate assembly 1k located in a main machine (not shown in the figure) are prevented from falling into the main machine through the pouring port 1112k. A housing 11k is closed to protect the main machine to a particular extent, the service life is prolonged, the polluted region is limited to be maintained in an accommodation space 102k, and diffusion of the polluted region is avoided.

Specifically, the shape of the ash collection member 16k is adapted to the shape of the pouring port 1112k. The ash collection member includes two walls connected to each other. The two walls correspond to the rear side wall portion and the right side wall portion, penetrated by the pouring port 1112k, of the first housing 111k in shape respectively.

It should be understood that the connection method between the ash collection member 16k and the first housing 111k is not limited herein. The ash collection member may be detachably connected to the first housing 111k through inserting, fastening, bolting, etc., or may be rotatably connected to the first housing 111k through a hinge, a rotary shaft, an integrally-formed flexible rotary connection portion, etc.

In some other optional embodiments, the size of the ash collection member 16k may be greater than that of the pouring port 1112k. The ash collection member covers the pouring port 1112k by covering the first housing 111k where the pouring port 1112k is located.

Further, the ash collection member 16k includes a body 161k and a collection portion 162k. The body 161k is used for covering the pouring port 1112k. The collection portion 162k is arranged on the body 161k and provided with a channel for the strip-shaped substrate 20k to pass through. The channel matches the strip-shaped substrate 20k in size, so that scraps (including scraps that have been separated from the strip-shaped substrate 20k, and scraps that are superficially attached to the surface of the strip-shaped substrate 20k and are likely to be separated) generated by the atomized strip-shaped substrate 20k can be stopped by the collection portion 162k at the channel, and are retained in the ash collection member 16k. Thus, the ash collection effect of the ash collection member 16k is improved, fewer scraps are separated in a process of winding the strip-shaped substrate 20k around the second reel 321k, and the scraps are retained in the accommodation space 102k.

Specifically, in the embodiment, the collection portion 162k is in a plate shape and in a cambered shape on two sides of the strip-shaped substrate 20k. A cambered opening of the collection portion is towards the third guide member 43k.

In some other optional embodiments, the collection portion 162k may alternatively be in a rectangular shape having an opening in one side or a funneled shape. The collection portion may be in any shape as long as the scraps can be collected.

In some embodiments, the body 161k may be further provided with the side wall. The side wall is at least arranged around a periphery of the portion, corresponding to the rear side wall portion penetrated by the pouring port 1112k of the first housing 111k, of the body 161k. Thus, the body 161k is approximately in a drawer shape, accommodation is achieved, and the scraps collected by the collection portion 162k are prevented from being separated from the ash collection member 16k.

FIG. 24 shows an atomization substrate assembly 1m in a twelfth embodiment of the present disclosure. The differences from the atomization substrate assembly 1f in the sixth embodiment are as follows:
In the embodiment, an included angle exists between the connection line between a first reel 311m and a second reel 321m and the axis (the axis in the length direction) of the housing 11m.

Specifically, with the angle shown in FIG. 24 as an example, the second reel 321m is approximately located at the upper left side of a housing space 100m, and the first reel 311m is approximately located at the lower right side of the housing space 100m.

The first reel 311m and the second reel 321m are arranged in a staggered manner in the housing space 100m, so that the utilization rate of the housing space 100m is increased, and the atomization substrate assembly 1m is further miniaturized.

In some other optional embodiments, the first reel 311m may alternatively be arranged at the lower left side of the housing space 100m, and the second reel 321m is approximately arranged at the upper right side of the housing space 100m. Thus, an included angle between the connection line from the second reel 321m to a third guide member 43m and the connection line from the third guide member 43m to a second guide member 42m is closer to 90 degrees, and an included angle between the connection line from the first reel 311m to the second guide member 42m and the connection line from the third guide member 43m to the second guide member 42m is also closer to 90 degrees. Further, neither of a first guide member 41m and a fourth guide member 44m is arranged, and the similar guiding effect of the strip-shaped substrate 20m in the housing space 100m can be achieved. To be specific, a number of members of the atomization substrate assembly 1m and the cost are reduced while miniaturization is achieved.

In the descriptions of the present disclosure, the descriptions with reference to the terms such as "an embodiment", "some embodiments", "an example" "a specific example", and "some examples" indicate that the specific features, structures, materials, or characteristics described with reference to the embodiment or the example are included in at least one embodiment or example of the present disclosure. In the present disclosure, the illustrative expressions of the above terms are not necessarily directed at the same embodiment or example. Moreover, the particular feature, structure, material or characteristic described can be combined in any suitable manner in any one or more embodiments or examples. In addition, without mutual contradiction, those skilled in the art can combine different embodiments or examples described in the present disclosure, and the features in different embodiments or examples.

What are described above are merely preferred embodiments of the present disclosure and are not used for limiting the present disclosure. Those skilled in the art can make various modifications and variations to the present disclosure. Any modification, equivalent replacement, improvement, etc. made within the spirit and principles of the present disclosure should fall within the scope of protection of the present disclosure.

## Claims

1. An atomization substrate assembly, comprising:
a strip-shaped substrate; and
a housing assembly, wherein the housing assembly comprises a housing, the housing is provided with a housing space for housing the strip-shaped substrate and comprises a first housing and a second housing, one of the first housing and the second housing is provided with an opening for placing the strip-shaped substrate into the housing space, and the other one of the first housing and the second housing is arranged on the opening in a covering manner.

2. The atomization substrate assembly of claim 1, wherein the housing assembly is provided with an atomization cavity, the housing space comprises a storage space and an accommodation space, and the storage space is spaced from the accommodation space;
the atomization substrate assembly further comprises a feeding assembly, and the feeding assembly comprises a first reel arranged in the storage space and a second reel arranged in the accommodation space; and
the outer peripheral side of the first reel is used for winding the strip-shaped substrate, the strip-shaped substrate is unwound from the first reel and wound around the outer peripheral side of the second reel after passing through the atomization cavity, and the strip-shaped substrate is atomized to generate an aerosol in the atomization cavity.

3. The atomization substrate assembly of claim 2, comprising at least one guide member used for guiding the strip-shaped substrate to move.

4. The atomization substrate assembly of claim 2, wherein the housing assembly comprises an atomization seat, the atomization seat is at least partially arranged in the housing and is provided with the atomization cavity and an air outlet channel communicating with the atomization cavity, and the air outlet channel is used for discharging the aerosol in the atomization cavity.

5. The atomization substrate assembly of claim 4, wherein the side wall of the side, in the thickness direction of the atomization substrate assembly, of the atomization seat is provided with a first avoiding port for the atomization assembly to extend, the portion, corresponding to the first avoiding port, of the housing is provided with a second avoiding port, and the second avoiding port is in butt joint with the first avoiding port.

6. The atomization substrate assembly of claim 4, wherein the housing is in a flat box shape, and the second housing and the first housing are in butt joint in the thickness direction and jointly define the storage space and the accommodation space; the feeding assembly is arranged at the bottom side of the atomization seat; the strip-shaped substrate moves in the atomization cavity in the transverse direction; and the transverse direction, the thickness direction, and the top-bottom direction are perpendicular to one another.

7. The atomization substrate assembly of claim 6, wherein the side, towards the first housing, of the atomization seat is opened and abuts against the inner surface of the first housing; the second housing is provided with a mounting port; and the side, farthest away from the first housing, of the atomization seat is arranged in the mounting port in a penetrating manner.

8. The atomization substrate assembly of claim 4, wherein the atomization seat comprises a seat housing and a raised post protruding along the top surface of the seat housing in the direction towards the top side, the atomization cavity is defined by the seat housing, the raised post penetrates out of the top end surface of the housing, and the air outlet channel is at least partially defined by a space in the raised post.

9. The atomization substrate assembly of claim 2, wherein the housing assembly further comprises a partition member, one end of the partition member is arranged at the inner surface of the first housing, and the other end extends in the thickness direction; the first housing and the second housing are in butt joint in the thickness direction and jointly define the storage space and the accommodation space along with the partition member; and the front end of the partition member at least forming the storage space is in sealing fit with the second housing.

10. The atomization substrate assembly of claim 9, comprising a first sealing member, wherein the first sealing member is sandwiched between the second housing and the front end of the partition member forming the storage space.

11. The atomization substrate assembly of claim 10, wherein the second housing is provided with a sealing recess, and the first sealing member is arranged in the sealing recess.

12. The atomization substrate assembly of claim 9, wherein the side wall of the storage space is provided with an outlet, and the portion, in the storage space, of the strip-shaped substrate moves out of the storage space through the outlet; and
the atomization substrate assembly comprises a second sealing member arranged at the outlet, the second sealing member is used for sealing the outlet, and the strip-shaped substrate and the second sealing member are in sealing fit and are movable relative to each other.

13. The atomization substrate assembly of claim 12, wherein the second sealing member is provided with a through recess, and the strip-shaped substrate is movably arranged the through recess in a penetrating manner and is in sealing fit with the recess wall of the through recess.

14. The atomization substrate assembly of claim 1, wherein the first housing is detachably or rotatably connected to the second housing.

15. The atomization substrate assembly of claim 2, further comprising a guide assembly used for guiding the strip-shaped substrate from the first reel to the atomization cavity and the second reel at one time; and the guide assembly comprises at least two guide members, and the portion, between the two guide members, of the strip-shaped substrate is arranged in the atomization cavity in a penetrating manner.

16. The atomization substrate assembly of claim 15, wherein the guide members and the feeding assembly are arranged on the first housing separately, and the positions, corresponding to the guide members, of the second housing are respectively provided with limiting structures used for preventing the strip-shaped substrate from being separated.

17. The atomization substrate assembly of claim 2, wherein the first housing or the second housing is further provided with at least one connection structure, and the connection structure comprises connection holes provided in correspondence to the first reel and/or the second reel.

18. The atomization substrate assembly of claim 2, wherein the first housing and/or the second housing are/is further provided with pouring ports/a pouring port, and the pouring port communicates with the accommodation space.

19. The atomization substrate assembly of claim 18, wherein the housing assembly further comprises an ash collection member, and the ash collection member is detachably or rotatably arranged on the pouring port in a covering manner.

20. The atomization substrate assembly of claim 2, comprising an atomization assembly arranged at the housing assembly, and the atomization assembly at least partially extends into the atomization cavity to be used for atomizing the strip-shaped substrate.

21. The atomization substrate assembly of claim 20, wherein the atomization assembly comprises a heating member, and the heating member extends into the atomization cavity to be in contact with the strip-shaped substrate or spaced from the strip-shaped substrate in parallel.

22. The atomization substrate assembly of claim 21, wherein the portion, extending into the atomization cavity, of the heating member corresponds to the portion, in the atomization cavity, of the strip-shaped substrate in the width direction and/or is centrally arranged in the atomization cavity in the length direction.

23. An aerosol generating device, comprising:
a main machine, wherein the main machine comprises a machine case, a driving assembly, and a power supply assembly, and the power supply assembly is arranged in the machine case; and
the atomization substrate assembly of any one of claims 2 to 22, wherein the atomization substrate assembly is detachably arranged in the machine case, and the driving assembly partially extends into the atomization substrate assembly to be at least in drive connection to a second reel.

24. The aerosol generating device of claim 23, wherein a housing assembly comprises an atomization seat, the atomization seat is at least partially arranged in a housing and is provided with an atomization cavity and an air outlet channel communicating with the atomization cavity, and the air outlet channel is used for discharging an aerosol in the atomization cavity;
the side wall of the side, in the thickness direction of the atomization substrate assembly, of the atomization seat is provided with a first avoiding port for an atomization assembly to extend, the portion, corresponding to the first avoiding port, of the housing is provided with a second avoiding port, and the second avoiding port is in butt joint with the first avoiding port; and
the main machine further comprises the atomization assembly, the atomization assembly comprises a heating member and a connection plate connected to the heating member, the heating member extends into the atomization cavity to be in at least partial contact with a strip-shaped substrate, and the connection plate closes the second avoiding port or the first avoiding port.

25. The aerosol generating device of claim 24, wherein the connection plate is detachably connected with the housing or the side wall of the atomization cavity; and/or
the atomization assembly comprises a connector electrically connected to the heating member, the connector is arranged at the side, facing away from the atomization cavity, of the connection plate, and the connector is used for being electrically connected to the power supply assembly.
